# FASCICULE DE BREVET EUROPEEN

(11) **EP 2 039 682 B1**
(45) Date de publication et mention de la délivrance du brevet: **04.08.2010**
(21) Numéro de dépôt: 08290885.6
(22) Date de dépôt: 19.09.2008
(51) Int. Cl.: C07D 207/09, C07D 209/42, C07D 209/52, C07D 217/26, C07D 233/36, C07D 271/08, A61K 31/385, A61K 31/40, A61K 31/403, A61K 31/404, A61K 31/4245, A61K 31/472, C07D 495/10

(54) **Sels d'addition d'inhibiteurs d'enzyme de conversion de l'angiotensine avec des acides donneurs de NO, leur procédé de préparation et les compositions pharmaceutiques les contenant**
Additionssalze von Hemmern des Angiotensin konvertierenden Enzyms mit NO abgebenden Säuren, Verfahren zu ihrer Herstellung und die sie enthaltenden pharmazeutischen Zusammensetzungen
Addition salts of inhibitors of the angiotensin-conversing enzyme with NO-donor acids, method for their preparation and pharmaceutical compositions containing them

(30) Priorité: 21.09.2007 FR 0706629
(43) Date de publication de la demande: 25.03.2009
(73) Titulaire: Les Laboratoires Servier, 92415 Courbevoie Cedex (FR)
(72) Inventeur: de Nanteuil, Guillaume, 92150 Suresnes (FR); Portevin, Bernard, 45450 Fay Aux Loges (FR); Gloanec, Philippe, 78160 Marly le Roi (FR); Parmentier, Jean-Gilles, 92130 Issy Les Moulineaux (FR); Benoist, Alain, 95130 Franconville (FR); Verbeuren, Tony, 78540 Vernouillet (FR); Rupin, Alain, 37510 Savonnieres (FR); Courchay, Christine, 91430 Igny (FR); Simonet, Serge, 78700 Conflans Sainte Honorine (FR)
(74) Mandataire: Giudicelli, Cathy

(56) Documents cités:
- EP-A- 0 575 754
- WO-A-00/12110
- WO-A-2004/050084
- WO-A-2006/078995
- WO-A-2006/102071
- WO-A-2007/059311
- WO-A-2007/060112
- US-A1- 2003 216 384
- DATABASE CA [Online] CHEMICAL ABSTRACTS SERVICE, COLUMBUS, OHIO, US; POKROVSKII, M. V. ET AL: "Method for correcting endothelial dysfunction" XP002487150 extrait de STN Database accession no. 2006:218242 & RU 2 271 202 C1 (RUSSIA) 10 mars 2006 (2006-03-10)
- PERSSON, K. ET AL: "Nitric oxide donors and angiotensin - converting enzyme inhibitors act in concert to inhibit human angiotensin - converting enzyme activity and platelet aggregation in vitro" EUROPEAN JOURNAL OF PHARMACOLOGY, vol. 406, no. 1, 2000, pages 15-23, XP002487146
- WANG, PENG GEORGE ET AL: "Nitric Oxide Donors: Chemical Activities and Biological Applications" CHEMICAL REVIEWS (WASHINGTON, D. C.), vol. 102, no. 4, 2002, pages 1091-1134, XP002487148
- STANISAVLJEVIC, SINISA ET AL: "Angiotensin I- converting enzyme inhibitors block protein kinase C.epsilon. by activating bradykinin B1 receptors in human endothelial cells" JOURNAL OF PHARMACOLOGY AND EXPERIMENTAL THERAPEUTICS, vol. 316, no. 3, 2006, pages 1153-1158, XP002487147
- DATABASE CA [Online] CHEMICAL ABSTRACTS SERVICE, COLUMBUS, OHIO, US; HAEFELI, WALTER E. ET AL: "Quinaprilat induces arterial vasodilation mediated by nitric oxide in humans" XP002487151 extrait de STN Database accession no. 1997:710123 & HYPERTENSION (DALLAS) , 30(4), 912-917 CODEN: HPRTDN; ISSN: 0194-911X, 1997,
- DATABASE CA [Online] CHEMICAL ABSTRACTS SERVICE, COLUMBUS, OHIO, US; RUIZ, FRANCISCO J. ET AL: "N-acetyl-L-cysteine potentiates depressor response to captopril and enalaprilat in SHRs" XP002487153 extrait de STN Database accession no. 1995:263273 & AMERICAN JOURNAL OF PHYSIOLOGY , 267(3, PT. 2), R767-R772 CODEN: AJPHAP; ISSN: 0002-9513, 1994,
- DATABASE CA [Online] CHEMICAL ABSTRACTS SERVICE, COLUMBUS, OHIO, US; MASSOUDY, P. ET AL: "Nitric oxide accounts for postischemic cardioprotection resulting from angiotensin - converting enzyme inhibition : indirect evidence for a radical scavenger effect in isolated guinea pig heart" XP002487152 extrait de STN Database accession no. 1995:408239 & JOURNAL OF CARDIOVASCULAR PHARMACOLOGY , 25(3), 440-7 CODEN: JCPCDT; ISSN: 0160-2446, 1995,
- SORBA, GIOVANNI ET AL: "Water Soluble Furoxan Derivatives as NO Prodrugs" JOURNAL OF MEDICINAL CHEMISTRY, vol. 40, no. 4, 1997, pages 463-469, XP002487149

## Description

La présente invention concerne de nouveaux sels d'addition d'inhibiteurs d'enzyme de conversion de l'angiotensine à des acides donneurs de NO, leur procédé de préparation et les compositions pharmaceutiques qui les contiennent.

Ces composés trouvent leur application dans le domaine de l'hypertension et des maladies cardiovasculaires.

L'hypertension entraîne un risque élevé d'accidents vasculaires, en particulier au niveau cérébral et coronaire. Elle est de plus en plus fréquemment associée à d'autres pathologies comme l'athérosclérose ou des maladies métaboliques telles que l'obésité, le diabète ou l'insuffisance rénale, ce qui augmente considérablement le risque de spasmes et de thromboses.

Depuis la découverte en 1980 de son action cardiovasculaire, le monooxyde d'azote (NO) est reconnu comme une molécule vasodilatatrice et vasoprotectrice capable de prévenir les vasospasmes, l'athérosclérose et la thrombose, ce médiateur offrant ainsi une protection contre les maladies cardiovasculaires. Le NO est essentiellement produit par l'endothélium. En général, le NO est reconnu d'avoir des propriétés vasodilatatrices, anti-adhésives, anti-thrombotiques, anti-inflammatoires et anti-oxydantes.
Les inhibiteurs de l'enzyme de conversion de l'angiotensine (IEC), comme le perindopril (Ferrari et al., 2005, Am J Hypertension, 18, 142S-154S), sont des agents thérapeutiques dont les effets protecteurs cardiovasculaires sont bien reconnus ; ces produits diminuent la pression artérielle, l'infarctus du myocarde, l'insuffisance cardiaque, la dysfonction ventriculaire gauche, le stroke et la mortalité cardiovasculaire. Ces effets bénéfiques sont également observés chez les patients diabétiques, avec ou sans athérosclérose. Pour le perindopril, les données cliniques récentes ont mis en évidence ses propriétés anti-athéromateuses et anti-inflammatoires et ses effets bénéfiques sur le dysfonctionnement endothélial (Ferrari et al., 2005). En bloquant l'enzyme de conversion, les IEC (1) préviennent la formation de l'angiotensine II, un puissant vasoconstricteur qui est impliqué dans les maladies cardiovasculaires (Kon et Jabs, 2004, Current Opinion Nephrol Hypertens, 13, 291-297 ; Unger, 2002, Am J Cardiol, 89, 3A-10A ; Ferrari et al., 2005) et (2) protègent la bradykinine de sa dégradation, la bradykinine dont les effets bénéfiques cardiovasculaires (par ex effets anti-ischémiques) sont dus à la production endothéliale de NO (Unger 2002 ; Ferrari et al., 2005).
Il est bien établi que dans plusieurs conditions pathologiques telles que l'athérosclérose, l'hypertension, le diabète et d'autres, la production de NO est diminuée voir même complètement absente (Gewaltig and Kodja, 2002 , Cardiovasc Res, 55, 250-260 ; Russo et al, 2002, Vasc Pharmacol, 38, 259-269). Dans cette condition de dysfonctionnement endothélial, les effets bénéfiques des IEC liés à l'inhibition de la dégradation de la bradykinine seront moins exprimés. Il a été postulé que des produits hybrides, inhibiteurs IEC et donneurs de NO pourraient être bénéfiques dans ces différentes conditions pathologiques, plus spécifiquement dans les pathologies cardiovasculaires.
De plus, des composés donneurs de NO, tels que la nitroglycérine, sont utilisés depuis longtemps pour soigner l'angine de poitrine et l'insuffisance cardiaque. L'effet bénéfique de ces produits est lié à leur capacité de former (de façon spontanée ou de façon métabolique) du NO. L'utilisation de ces produits a aussi conduit à l'observation que chez le sujet hypertendu, ces donneurs de NO provoquent une baisse prédominante de la pression artérielle systolique (Nesbitt, 2005, Hypertension, 45, 352-353). Une pression artérielle systolique non controlée est un facteur de risque important pour les accidents cérébraux et cardiaques et elle est souvent résistante aux traitements des anti-hypertenseurs. En effet, malgré des effets démontrés antihypertenseurs et vasoprotecteurs des produits tels que les IEC et d'autres classes de produits anti-hypertenseurs, la pression artérielle, en particulier systolique, reste difficile à contrôler et le taux de morbidité et mortalité reste élevé chez les sujets hypertendus.
Ajouter donc à des IEC tels que le perindopril, des propriétés donneurs de NO apparaît comme une stratégie importante dans la lutte contre les maladies cardiovasculaires. En effet, avoir des propriétés donneurs de NO améliorerait les propriétés antihypertensives, cardio-et vasculoprotectrices, le NO étant une molécule vasodilatatrice, anti-plaquettaire (donc anti-thrombotique), anti-adhésive et anti-oxydante (Waldorf et al. 2003, J Thromb Haemost, 1, 2112-2118 ; Gewaltig and Kodja, 2002, Cardiovasc Res, 55, 250-260).

Les composés de la présente invention présentent cette double activité pharmacologique leur conférant des propriétés intéressantes dans le domaine de l'hypertension et des pathologies cardiovasculaires.

Plus spécifiquement, la présente invention concerne les sels de formule (I) :

A • B (I)

dans laquelle A représente le perindopril ou le perindoprilate, B est un composé comportant au moins une fonction acide salifiable représenté par la formule (III) suivante :

X-(Ak₁)ₓ-(Y)_{y}-(Ak₂)_{z}-Z (III)

dans laquelle :
- X représente un groupement CO₂H, SO₃H ou P(O)(OH)₂,
- Ak₁ et Ak₂, identiques ou différents, représentent chacun un groupement alkylène C₁-C₈ linéaire ou ramifié, saturé ou insaturé, dans lequel un ou plusieurs des atomes de carbone peuvent être remplacés par un atome d'oxygène, de soufre, d'azote ou un groupement SO₂, ledit groupement alkylène étant éventuellement substitué par un ou plusieurs groupements choisis parmi CO₂H, SO₃H, hydroxy et amino,
- x, y et z, identiques ou différents, représentent chacun 0 ou 1,
- Y représente CO ou CONH,
- Z représente un groupement donneur de NO choisi parmi les groupements suivants 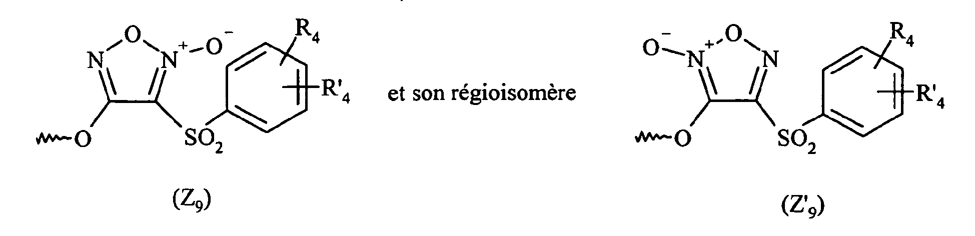 où R₄ et R'₄, identiques ou différents, représentent chacun un atome d'hydrogène ou d'halogène ou un groupement alkyle C₁-C₆ linéaire ou ramifié, trifluorométhyle ou trifluorométhoxy,
étant entendu que la liaison entre A et B est de type ionique.

Par groupement donneur de NO, on entend un groupement susceptible de donner, libérer et/ou transférer du monoxyde d'azote ou de l'une de ses formes biologiquement actives, et/ou de stimuler in vivo la production endogène de monoxyde d'azote ou de l'une de ses formes biologiquement actives.

Le perindopril et le perindoprilate sont représentés respectivement par les formules (IId) et (IIe) :

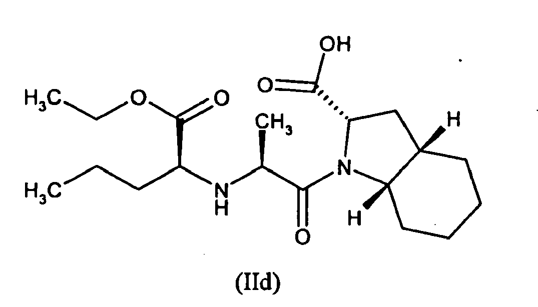

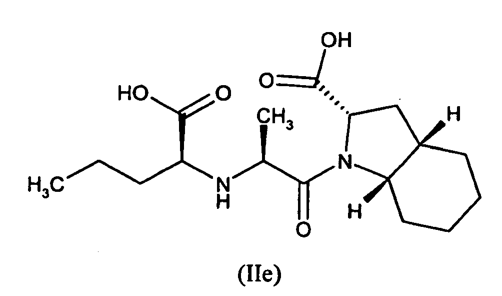

Parmi les composés de formule (III), on peut citer les composés appartenant à l'une des formules suivantes :

HO₂C-(Ak₁)ₓ-Z (IIIa)

HO₂C-CH=CH-(Ak₃)ₓ-Z (IIIb)

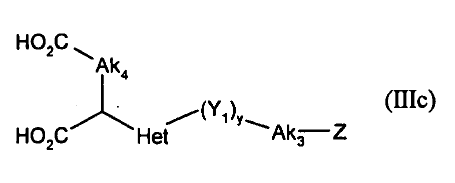

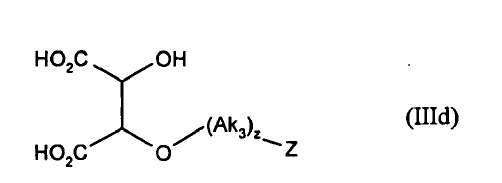

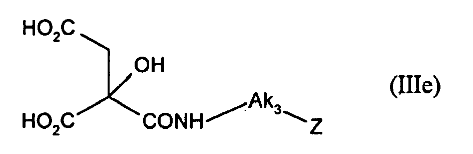

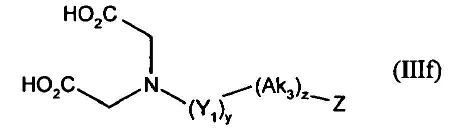

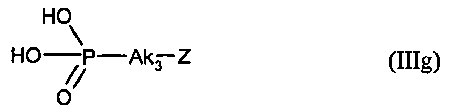

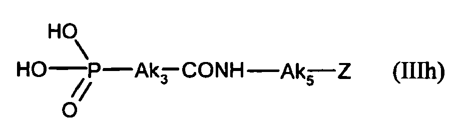

HO₃S-Ak₆CONH-Ak₅-Z (IIIi)

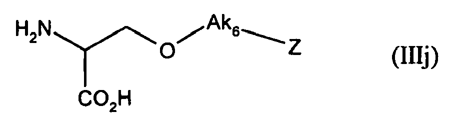

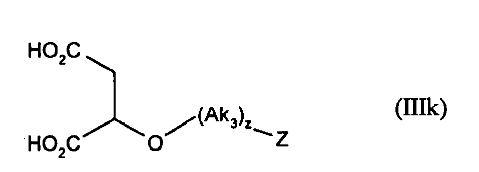

dans lesquelles :
Ak₁, x, y, z et Z sont tels que définis précédemment,
Ak₃ et Ak₅, identiques ou différents, représentent chacun un groupement alkylène C₁-C₆ linéaire ou ramifié saturé dans lequel un ou plusieurs des atomes de carbone peuvent être remplacés par un atome d'oxygène, de soufre, d'azote ou un groupement SO₂,
Ak₄ représente un groupement alkylène C₁-C₃ linéaire ou ramifié saturé dans lequel un des atomes de carbone peut être remplacé par un atome d'oxygène, de soufre, d'azote ou un groupement SO₂,
Ak₆ représente un groupement alkylène C₂-C₅ linéaire ou ramifié saturé dans lequel un des atomes de carbone peut être remplacé par un atome d'oxygène, de soufre, d'azote ou un groupement SO₂,
Het représente O ou NH,
et Y₁ représente un groupement carbonyle.

Les composés B préférés sont les suivants :

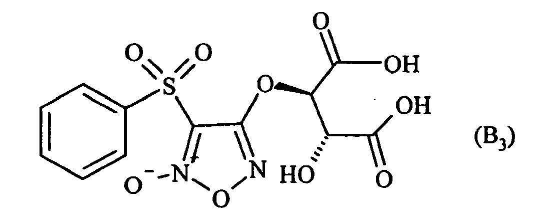

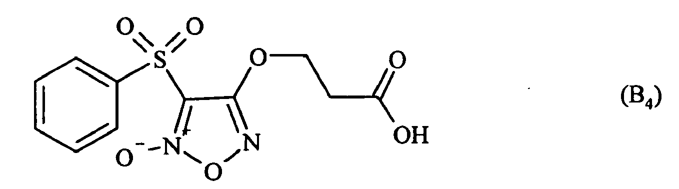

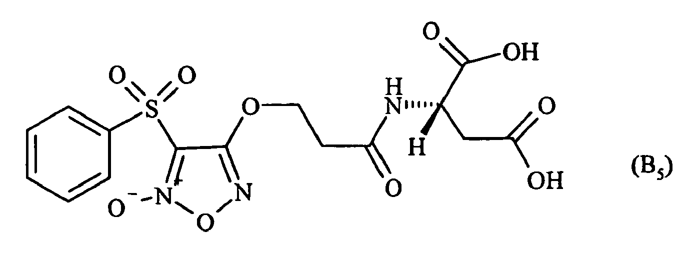

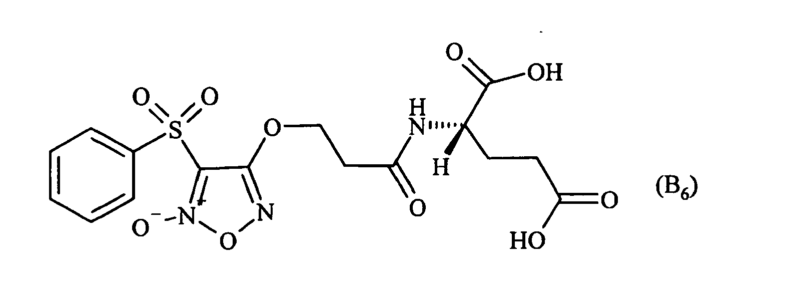

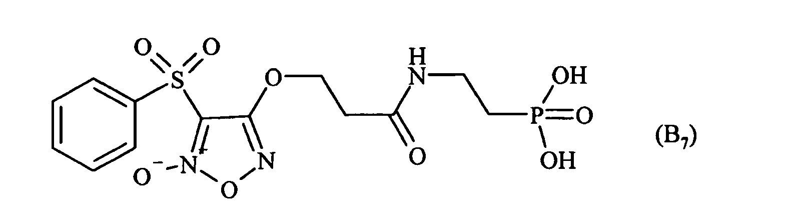

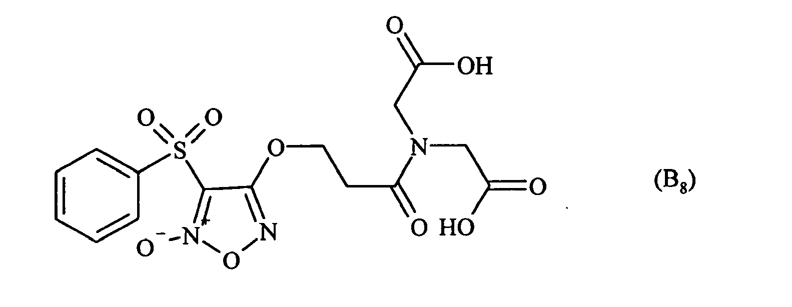

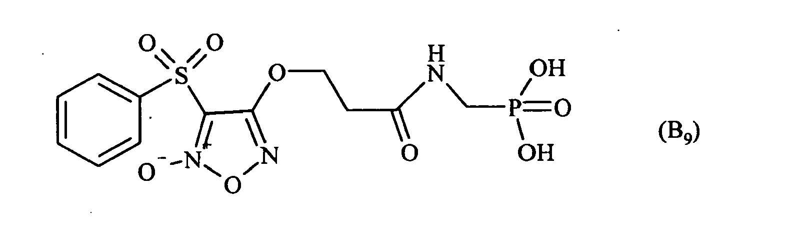

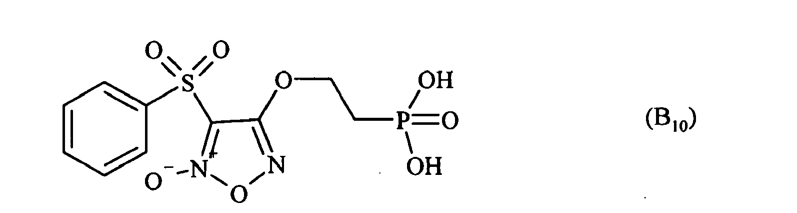

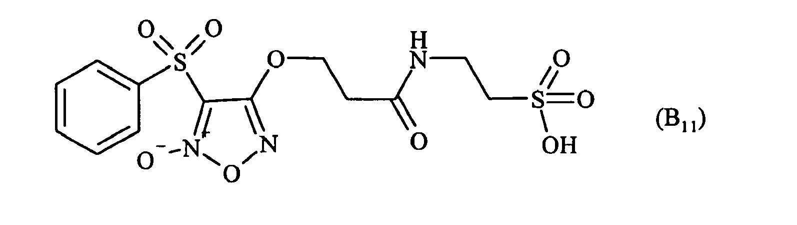

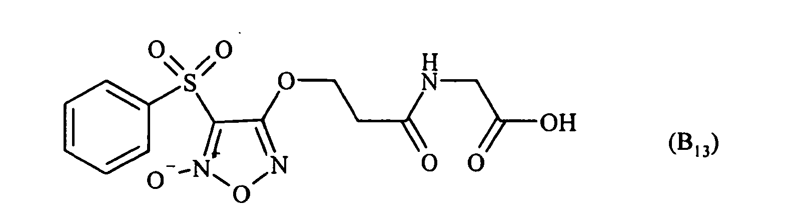

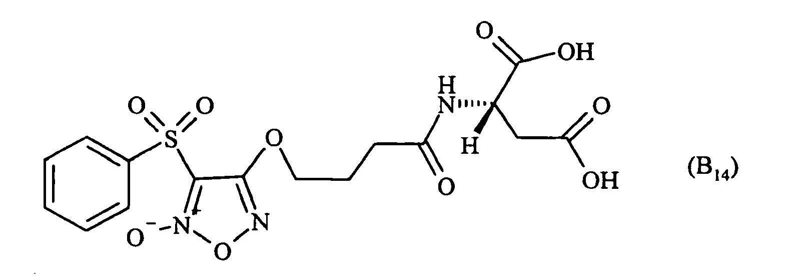

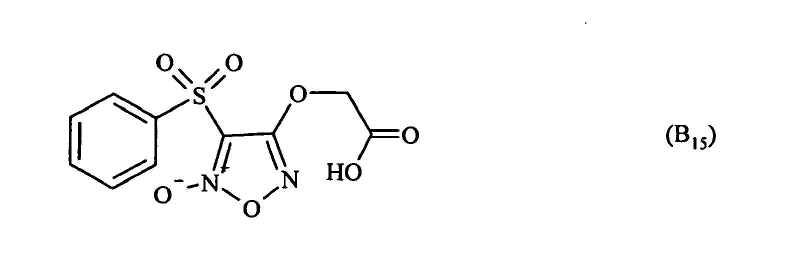

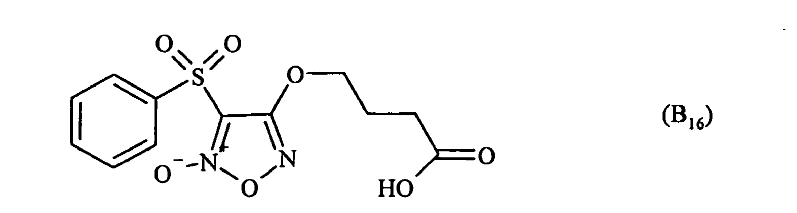

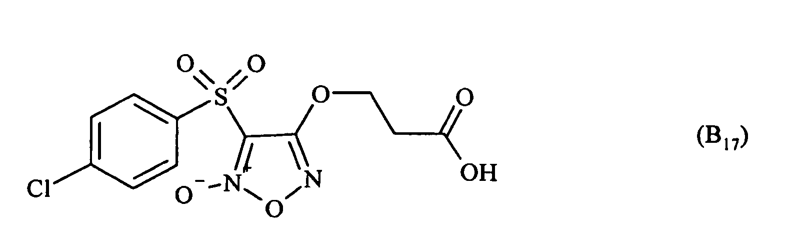

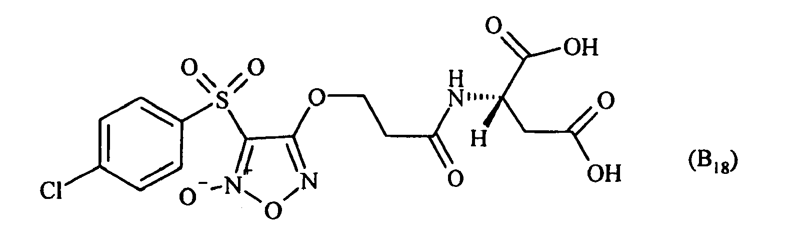

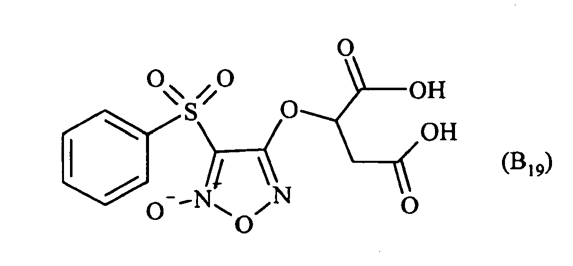

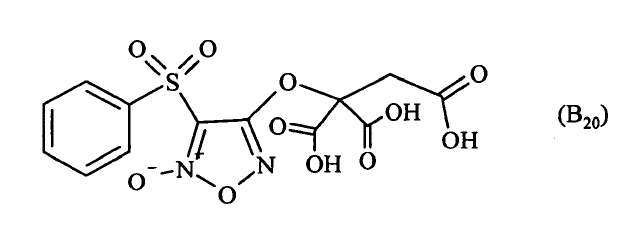

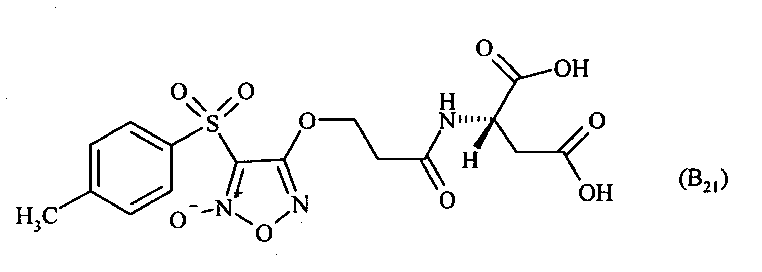

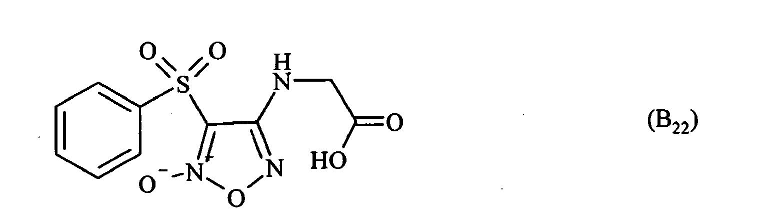

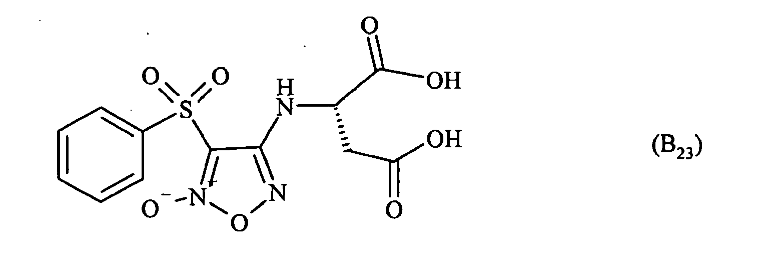

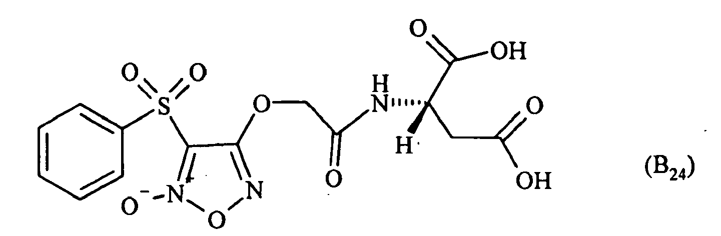

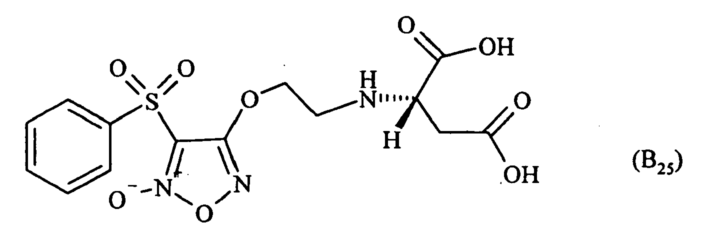

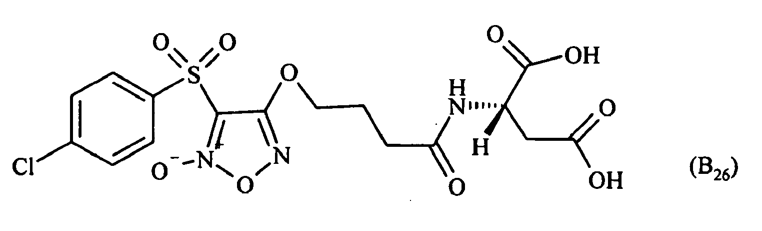

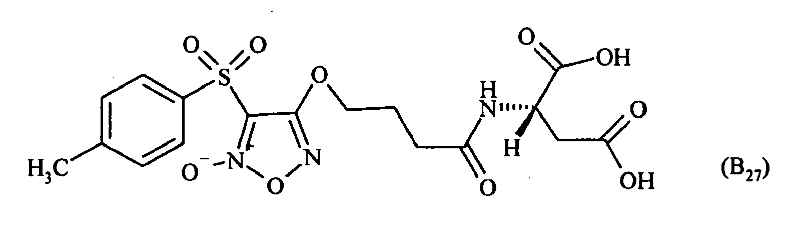

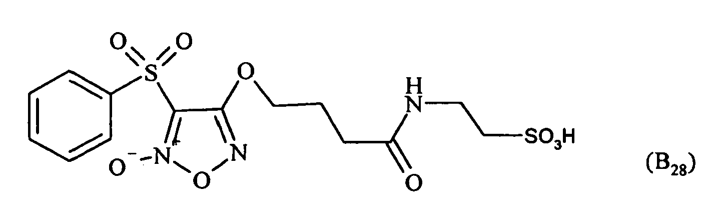

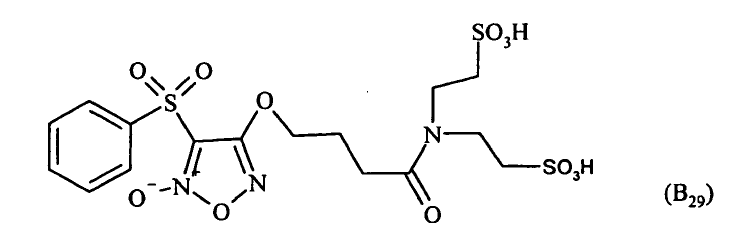

Les composés préférés de formule (I) sont les suivants :
- Acide (2*S*)-2-[(3-{[5-oxydo-4-(phénylsulfonyl)-1,2,5-oxadiazol-3-yl]-oxy}-propionyl)-amino]-succinique-Acide (2S,3aS,7aS)-1-[(2S)-2-{[(1S)-1-(éthoxycarbonyl)-butyl]-amino}-propionyl]-octahydro-1H-indole-2-carboxylique (1:1);
- Acide (2*S*)-2-[(3-{[5-oxydo-4-(phénylsulfonyl)-1,2,5-oxadiazol-3-yl]-oxy}-propionyl)-amino]-pentanedioique - Acide (2*S*,3a*S*,7a*S*)-1-[(2*S*)-2-{[(1*S*)-1-(éthoxycarbonyl)-butyl]-amino}-propionyl]-octahydro-1*H-*indole-2-carboxylique (1:1);
- Acide 2-[(3-{[5-oxydo-4-(phénylsulfonyl)-1,2,5-oxadiazol-3-yl]-oxy}-propionyl)-amino]-éthanesulfonique - Acide (2*S*,3a*S*,7a*S*)-1-[(2*S*)-2-{[(1*S*)-1-(éthoxycarbonyl)-butyl]-amino}-propionyl]-octahydro-1*H-*indole-2-carboxylique (1:1);
- Acide (2*S*,3a*S*,7a*S*)-1-[(2*S*)-2-{[(1*S*)-1-(éthoxycarbonyl)-butyl]-amino}-propionyl]-octahydro-1*H-*indole-2-carboxylique - Acide {[(3-{[5-oxydo-4-(phénylsulfonyl)-1,2,5-oxadiazol-3-yl]-oxy}-propionyl)-amino]-méthyl}-phosphonique (1:1);
- Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique - Acide 2,2'-[(3-{[5-oxydo-4-(phénylsulfonyl)-1,2,5-oxadiazol-3-yl]-oxy}-propionyl)-imino]-diacétique (1:1);
- Acide (2*S*,3a*S*,7a*S*)-1-[(2*S*)-2-{[(1*S*)-1-(éthoxycarbonyl)-butyl]-amino}-propionyl]-octahydro-1*H-*indole-2-carboxylique-Acide {2-[(3-{[5-oxydo-4-(phénylsulfonyl)-1,2,5-oxadiazol-3-yl]-oxy}-propionyl)-amino]-éthyl}-phosphonique (1:1);
- Acide (2*S*,3a*S*,7a*S*)-1-[(2*S*)-2-{[(1*S*)-1-(éthoxycarbonyl)-butyl]-amino}-propionyl]-octahydro-1*H-*indole-2-carboxylique - Acide (2-{[5-oxydo-4-(phénylsulfonyl)-1,2,5-oxadiazol-3-yl]-oxy}-éthyl)-phosphonique (1:1).;
- Acide (2S)-2-[(4-{[5-oxydo-4-(phénylsulfonyl)-1,2,5-oxadiazol-3-yl]-oxy}-butanoyl)-amino]-succinique- Acide (2*S*,3a*S*,7a*S*)-1-[(2*S*)-2-{[(1*S*)-1-(éthoxycarbonyl)-butyl]-amino}-propionyl]-octahydro-1H-indole-2-carboxylique (1:1)
- Acide (2S)-2-{[3-({4-[(4-chlorophényl)sulfonyl]-5-oxydo-1,2,5-oxadiazol-3-yl}oxy)propionyl]amino}succinique- Acide (2S,3aS,7aS)-1-[(2S)-2-{[(1S)-1-(éthoxycarbonyl)-butyl]-amino}-propionyl]-octahydro-1H-indole-2-carboxylique (1:1);
- Acide (2S)-2-{[3-({4-[(4-méthylphényl)sulfonyl]-5-oxydo-1,2,5-oxadiazol-3-yl}oxy)propionyl]amino}succinique - Acide (2S,3aS,7aS)-1-[(2S)-2-{[(1S)-1-(éthoxycarbonyl)-butyl]-amino}-propionyl]-octahydro-1*H-*indole-2-carboxylique (1:1);
- Acide (2S)-2-{[3-({4-[(4-méthylphényl)sulfonyl]-5-oxydo-1,2,5-oxadiazol-3-yl}oxy)butanoyl]amino}succinique - Acide (2S,3aS,7aS)-1-[(2S)-2-{[(1*S*)-1-(éthoxycarbonyl)-butyl]-amino}-propionyl]-octahydro-1*H-*indole-2-carboxylique (1:1).

La présente invention concerne également un procédé de synthèse des composés de formule (I), dans lequel le composé A est mis en réaction avec le composé B en quantité au moins égale à 1 équivalent du composé A.

Les composés A de formules (IId) et (IIe) sont connus.

Les composés B peuvent être obtenus par des réactions classiques de la chimie organique.

Les composés B de formule (III) peuvent être obtenus par réaction entre le composé de formule suivante : 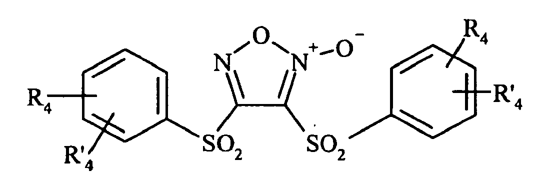 et un dérivé de l'alcool X-(Ak₁)ₓ-(Y)_{y}-(Ak₂)_{z}-OH dont les fonctions acides sont protégées, en présence de base, suivie de la déprotection des fonctions acides.

Les composés B de formule (III) sont des produits nouveaux, utiles comme intermédiaires de synthèse dans l'industrie chimique ou pharmaceutique, notamment dans la synthèse des sels de formule (I), et font à ce titre partie intégrante de la présente invention.

Les composés de la présente invention, de par leurs propriétés pharmacologiques sont indiqués dans les pathologies qui nécessitent un traitement par un IEC et/ou par un donneur de NO et sont utiles dans le traitement des pathologies cardiovasculaires telles que l'hypertension artérielle (dans ses différentes formes) et ses conséquences vasculaires et rénales, l'hypertension systolique, les maladies vasculaires périphériques, l'athérosclérose, la resténose, l'insuffisance cardiaque, la thrombose et tous les événements thromboemboliques, l'angine de poitrine (stable ou instable), les accidents vasculaires cérébraux, les accidents coronariens, l'infarctus du myocarde, le remodelage vasculaire, le diabète et ses conséquences vasculaires et rénales, les complications liées aux interventions chirurgicales tels que les chirurgies cardiovasculaires et la dysfonction endothéliale.
Du fait que le NO présente également des propriétés anti-inflammatoires et anti-oxydantes, les composés de la présente invention sont utiles dans le traitement des pathologies impliquant l'inflammation et le stress oxydant.

La présente invention a également pour objet les compositions pharmaceutiques contenant comme principe actif un composé de formule (I), en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

Outre le composé de formule (I), les compositions pharmaceutiques selon l'invention contiennent un ou plusieurs excipients ou véhicules tels que des diluants, des lubrifiants, des liants, des agents de désintégration, des absorbants, des colorants, des édulcorants.

A titre d'exemple d'excipients ou véhicules, on peut citer :
◆ *pour les diluants :* le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

Le pourcentage de principe actif de formule (I) dans la composition pharmaceutique est préférentiellement compris entre 5% et 50% en poids.

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent la présente invention. Les structures des composés décrits dans les exemples ont été déterminées selon les techniques spectrophotométriques usuelles (infrarouge, résonance magnétique nucléaire, spectrométrie de masse).

### Abréviations :

- DCC: N,N'-dicyclohexylcarbodiimide
- DIEA: N,N-diisopropyléthylamine
- DMAP: 4-diméthylaminopyridine
- DMF: diméthylformamide
- EDCI: 1-(3-diméthylaminopropyl)-3-éthylcarbodiimide (chlorhydrate)
- HOBT: 1-hydroxybenzotriazole
- THF: tétrahydrofurane
- TFA: acide trifluoroacétique

### EXEMPLE 1 : Acide (2S)-2-[(3-{[5-oxydo-4-(phénylsulfonyl)-1,2,5-oxadiazol-3-yl]-oxy}-propionyl)-amino]-succinique-Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : 3-[(5-Oxydo-4-(phénylsulfonyl)-1,2,5-oxadiazol--3-yl)-oxy]-propionate de terbutyle

A une solution refroidie à 15°C de 3,4-bis(phénylsulfonyl)-1,2,5-oxadiazole 2-oxyde (16 g- 44 mmol) et de 3-hydroxypropionate de terbutyle (10g-68 mmol) dans 200 ml de THF anhydre est additionnée en 15 minutes une solution aqueuse de NaOH à 50% (6,4g-80 mmol). Après 2h d'agitation le solvant est éliminé sous vide et le résidu repris dans 100 ml d'eau et 100 ml d'acétate d'éthyle. La phase organique est décantée, lavée avec une solution saturée de NaCl, séchée par du sulfate de sodium et évaporée.
L'huile résiduelle est purifiée par flash chromatographie, en utilisant comme éluant un mélange dichlorométhane / acétate d'éthyle (98/2) pour conduire au produit attendu.

### Stade B : Acide 3-[(5-oxydo-4-(phénylsulfonyl)-1,2,5-oxadiazol--3-yl)-oxy]-propionique (composé B₄)

14 g de composé obtenu lors du stade A, en solution dans 100 ml de dichlorométhane sont placés sous argon, additionnés de 10 ml de TFA puis agités 6 heures à température ambiante.
On élimine les solvants sous vide. Le résidu est cristallisé dans l'éther diisopropylique pour conduire au produit attendu.
*Point de fusion :* 138°C.

### Stade C : (2S)-2-[(3-{[5-Oxydo-4-(phénylsulfonyl)-1,2,5-oxadiazol-3-yl]-oxy}-propionyl)-amino]-succinate de diterbutyle.

Une solution du composé obtenu au stade précédent (2,7g-8 mmol), de L-aspartate de diterbutyle chlorhydrate (2,25 g- 8 mmol), EDCI (1,62 g-8 mmol), DIEA (1,32g-8mmol) et HOBT (1,1g-8mmol) dans 100 ml de DMF anhydre est agitée 72h à température ambiante sous argon. Le DMF est éliminé par distillation puis le résidu est repris dans 100 ml d'eau et 100 ml d'acétate d'éthyle. La phase organique est ensuite lavée par une solution de NaHCO₃ à 5%, séchée sur Na₂SO₄ puis évaporée à sec. On purifie le brut réactionnel par flash chromatographie en éluant par un mélange dichlorométhane/acétate d'éthyle (90:10).

### Stade D : Acide (2S)-2-[(3-{[5-oxydo-4-(phénylsulfonyl)-1,2,5-oxadiazol-3-yl]-oxy}-propionyl)-amino]-succinique (composé B₅).

On agite une solution du composé obtenu lors du stade précédent (4 g-7,3 mmol) dans 100ml de dichlorométhane et 15 ml de TFA pendant 6 heures sous argon. On distille les solvants à sec. On triture le résidu dans l'éther diisopropylique pendant 2h jusqu'à cristallisation. Essorer, sécher.
*Point de fusion :* 130-131°C.

### Stade E : Acide (2S)-2-[(3-{[5-oxydo-4-(phénylsulfonyl)-1,2,5-oxadiazol-3-yl]-oxy}-propionyl)-amino]-succinique- Acide (2S,3aS, 7aS)-1-[(2S)-2-{[(1S)-1-(éthoxycarbonyl)-butyl]-amino}-propionyl-octahydro-1H-indole-2-carboxylique (1:1)

On prépare une solution de perindopril ou acide (2S,3aS,7aS)-1-[(2S)-2-{[(1S)-1-(éthoxycarbonyl)-butyl]-amino}-propionyl]-octahydro-1H-indole-2-carboxylique (368 g-1 mmol) et du composé obtenu au stade précédent (429 g-1 mmol) dans 50 ml d'eau et 50 ml d'acétonitrile. Filtrer sur Whatman et lyophiliser.

| *Microanalyse élémentaire :* | | | | |
|---|---|---|---|---|
| | *%C* | *%H* | *%N* | *%S* |
| *Calculé* | *51,19* | 5*,94* | *8,78* | *4, 02* |
| *Trouvé* | *51,49* | *5,79* | *8,80* | *3,70* |

### EXEMPLE 3 : Acide (2R,3R)-2-hydroxy-3-{[5-oxydo-4-(phénylsulfonyl)-1,2,5-oxadiazol-3-yl]-oxy}-succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : (2R,3R)-2-hydroxy-3-{[5-oxydo-4-(phénylsulfonyl)-1,2,5-oxadiazol-3-yl]-oxy}-succinate de diterbutyle

A une solution de 3,4-bis(phénylsulfonyl)-1,2,5-oxadiazole 2-oxyde (7g-19 mmol) et de tartrate de diterbutyle (5g - 19 mmol) dans 100 ml de THF anhydre, on additionne une solution de NaOH à 50% (1,2g-30 mmol) en 1/2h. Après 5h d'agitation le solvant est évaporé à sec et le résidu repris dans eau et acétate d'éthyle. La fraction organique est séchée au Na₂SO₄ et évaporée à sec puis purifiée par flash chromatographie en éluant par un mélange de dichlorométhane/éthanol (98:2), pour conduire au produit attendu sous la forme d'une huile incolore.

### Stade B : Acide (2R,3R)-2-hydroxy-3-{[5-oxydo-4-(phénylsulfonyl)-1,2,5-oxadiazol-3-yl}-oxy}-succinique (composé B₃)

Le composé obtenu au stade précédent est agité 4h dans 50ml de dichlorométhane et 10ml de TFA.
Les solvants sont éliminés sous vide et le résidu est cristallisé dans l'éther diisopropylique. *Point de fusion :* 198-200°C.

### Stade C : Acide (2R,3R)-2-hydroxy-3-{[5-oxydo-4-(phénylsulfonyl)-1,2,5-oxadiazol-3-yl]-oxy}-succinique - Acide (2S,3aS,7aS)-1-[(2S)-2-{[(1S)-1-(éthoxycarbonyl)-butyl)-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | *50,13* | 5*,70* | *7,54* | *4,32* |
| *Trouvé* | *49,68* | *5,30* | *7,74* | *4,17* |

### EXEMPLE 4 : Acide (2S)-2-[(3-{[5-oxydo-4-(phénylsulfonyl)-1,2,5-oxadiazol-3-yl]-oxy}-propionyl)-amino]-pentanedioique - Acide (2S,3aS,7aS)-1-[(2S)-2-{([(1S)-1-(éthoxycarbonyl)-butyl]-amino}-propionyl]-octahydro-1H-indole-2-carboxylique (1:1)

### Stade A : (2S)-2-[(3-{[5-oxydo-4-(phénylsulfonyl)-1,2,5-oxadiazol-3-yl]-oxy}-propionyl)-amino]-pentanedioate de diterbutyle

Une solution préparée à partir du composé B4 obtenu au stade B de l'Exemple 1 (2,5 g-8mmol), de L-glutamate de diterbutyle-chlorhydrate (2,36g-8mmol), de EDCI (1,62g-8mmol), de DIEA (1,32ml-8mmol) et de HOBT (1,1g- 8mmol) dans 100 ml de DMF anhydre est agitée 72h à température ambiante. Le DMF est éliminé par distillation puis le résidu est repris dans 100 ml d'eau et 100 ml d'acétate d'éthyle. La phase organique est lavée par une solution de NaHCO₃ à 5% puis par de l'eau. On sèche sur Na₂SO₄ et évapore à sec. On purifie par flash chromatographie en éluant par un mélange dichlorométhane/acétate d'éthyle (90:10) pour conduire au produit attendu sous la forme d'une huile incolore.

### Stade B : Acide (2S)-2-[(3-{[5-oxydo-4-(phénylsulfonyl)-1,2,5-oxadiazol-3-yl]-oxy}-propionyl)-amino]-pentanedioique (composé B₆)

On agite une solution du composé obtenu lors du stade A (3g-5,5mmol) dans 100ml dichlorométhane et 15ml de TFA pendant 6h sous argon. On distille les solvants sous vide. Le résidu est trituré dans l'éther diisopropylique, essoré puis séché sous vide pour conduire au produit attendu sous la forme de cristaux blancs.
*Point de fusion :* 157-158°C.

### Stade C : Acide (2S)-2-[(3-{[5-oxydo-4-(phénylsulfonyl)-1,2,5-oxadiazol-3-yl]-oxy}-propionyl)-amino]pentanedioique - Acide (2S,3aS,7aS)-1-[(2S)-2-{[(1S)-1-(éthoxycarbonyl)-butyl]-amino}propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | *51,78* | *6, 08* | *8, 63* | *3,95* |
| *Trouvé* | *51, 21* | *5,93* | *8, 69* | *3, 68* |

### EXEMPLE 6 : Acide 2-[(3-{[5-oxydo-4-(phénylsulfonyl)-1,2,5-oxadiazol-3-yl]-oxy}-propionyl)-amino]-éthanesulfonique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : N-{2-[(3-{[5-oxydo-4-(phénylsulfonyl)-1,2,5-oxadiazol-3-yl]-oxy}-propionyl)-oxy]}- 5-norbornène-2,3-dicarboximide

Une solution du composé B4 obtenu au stade B de l'Exemple 1 (2,5 g - 5,4 mmol), de N-hydroxy-5-norbornène-2,3-dicarboximide (1,03 g- 5,4 mmol) et de DCC (1,17g-5,4mmol) dans 100 ml de THF anhydre est agitée 20 heures à température ambiante. La dicyclohexylurée formée est éliminée par filtration et le THF est éliminé par distillation sous vide.
On reprend le produit brut dans 80 ml d'acétate d'éthyle, filtre à nouveau et évapore à sec. L'ester activé ainsi obtenu est utilisé sans purification au stade B.

### Stade B : Acide 2-[(3-{[5-oxydo-4-(phénylsulfonyl)-1,2,5-oxadiazol-3-yl]-oxy}-propionyl)-amino]-éthanesulfonique (composé B₁₁)

On agite une solution de l'ester activé obtenu au stade précédent (2,56 g- 5,4 mmol), de taurine (0,81 g - 6,5 mmol), et de NaHCO₃ (0,54 g - 6,5 mmol) dans 80 ml d'eau à température ambiante pendant 48h. Les solvants sont éliminés par distillation sous vide. Les résidus sont repris dans 100 ml d'eau et 100 ml d'acétate d'éthyle. Après extraction et décantation, la phase aqueuse est éluée sur une colonne de résine DOWEX 50WX8 échangeuse de cations. Le solvant est evaporé sous vide. On purifie le résidu sur une colonne préparative HPLC phase inverse éluée par un mélange eau-acétonitrile-TFA (650:350:1). Les fractions pures sont lyophilisées.

### Stade C : Acide 2-[(3-{[5-oxydo-4-(phénylsulfonyl)-1,2,5-oxadiazol-3-yl]-oxy}-propionyl)-amino]-éthanesulfonique - Acide (2S,3aS,7aS)-1-[(2S)-2-{[(1S)-1-(éthoxycarbonyl)-butyl-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *48, 66* | *6, 00* | *8,87* | *8,12* |
| *Trouvé* | *48,32* | *6,32* | *8, 66* | *8, 08* |

### EXEMPLE 7 : Acide (2S,3aS,7aS)-1-[(2S)-2-{[(1S)-1-(éthoxycarbonyl)-butyl]-amino}-propionyl]-octahydro-1H-indole-2-carboxylique-Acide {[(3-{[5-oxydo-4-(phénylsulfonyl)-1,2,5-oxadiazol-3-yl]-oxy}-propionyl)-amino]-méthyl}-phosphonique (1:1)

### Stade A : Acide {[(3-{[5-oxydo-4-(phény/sulfony/)-1,2,5-oxadiazo/-3-y/]-oxy}-propiony/)-amino]-méthyl}-phosphonique (composé B₉)

Une solution préparée à partir du composé obtenu au stade A de l'Exemple 6 (2,14g-4,5 mmol), de l'acide aminométhylphosphonique (0,7g-6 mmol) et de NaHCO₃ (1g-12 mmol) dans 50 ml de dioxane et 50 ml d'eau est agitée 72h à température ambiante.
Les solvants sont alors évaporés à sec et le résidu repris dans 100 ml d'eau et 100 ml d'acétate d'éthyle et agité 1 heure. Après décantation, on purifie la phase aqueuse sur une colonne de résine DOWEX 50WX8 échangeuse de cations. Le résidu obtenu après évaporation de l'eau est cristallisé dans l'acétonitrile, essoré, puis séché sous vide pour conduire au produit attendu.
*Point de fusion :* 171-172°C.

### Stade B : Acide (2S,3aS, 7aS)-1-[(2S)-2-{[{1S)-1-(éthoxycarbony/)-buty/]-amino}-propionyl]-octahydro-1H-indole-2-carboxylique -Acide {[(3-{[5-oxydo-4-(phénylsulfonyl)-1,2,5-oxadiazol-3-yl]-oxy}-propionyl)-amino]-méthyl}-phosphonique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *48, 00* | *5,98* | *9, 03* | *4,13* |
| *Trouvé* | *47,38* | *6,05* | *9, 07* | *3,97* |

### EXEMPLE 8 : Acide (2S,3aS,7aS)-1-[(2S)-2-{[(1S)-1-(éthoxycarbonyl)-butyl]-amino}-propionyl]-octahydro-1H-indole-2-carboxylique-Acide 2,2'-[(3-{[5-oxydo-4-(phénylsulfonyl)-1,2,5-oxadiazol-3-yl]-oxy}-propionyl)-imino]-diacétique (1:1)

### StadeA : 2,2'-[(3-{[5-oxydo-4-(phénylsulfonyl)-1,2,5-oxadiazol-3-yl]-oxy}-propionyl)-amino]-diacétate de terbutyle

Une solution du composé obtenu au stade B de l'Exemple 1 (3,5 g - 11 mmol), d'iminodiacétate de terbutyle (2,74 g - 11 mmol), HOBT (1,5 g - 11 mmol) et de EDCI (2,4 g - 12 mmol) dans 100 ml de DMF anhydre est agitée à température ambiante pendant 72h.
Le DMF est éliminé par distillation sous vide. Le résidu est repris dans 100 ml d'eau et 100 ml d'acétate d'éthyle. La phase organique est lavée par une solution aqueuse de NaHCO₃ à 5%, séchée sur Na₂SO₄ et évaporée à sec. On purifie le résidu par flash chromatographie en éluant par un mélange dichlorométhane-acétate d'éthyle (95:5) pour conduire au produit attendu sous la forme d'une huile incolore.

### Stade B : Acide 2,2'-[(3-{[5-oxydo-4-(phénylsulfonyl)-1,2,5-oxadiazol-3-yl]-oxy}-propionyl)-amino]-diacétique (composé B₈)

Une solution du composé obtenu au stade précédent (800 mg - 1,5 mmol) dans 50 ml de dichlorométhane et 5 ml de TFA est agitée 6h à température ambiante. Les solvants sont alors évaporés sous vide. Le résidu est cristallisé dans l'éther diisopropylique, essoré, puis séché sous vide de pompe pour conduire au produit attendu sous la forme de cristaux blancs.
*Point de fusion :* 154°C (décomposition).

### Stade C : Acide (2S,3aS,7aS)-1-[(2S)-2{[(1S)-1-(éthoxycarbonyl)-butyl]-amino}-propionyl]-octahydro-1H-indole-2-carboxylique - Acide 2,2'-[(3-{(5-oxydo-4-(phénylsulfonyl)-1,2,5-oxadiazol-3-yl]-oxy}propionyl)-imino]-diacétique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *51,19* | *5,94* | *8,78* | *4,02* |
| *Trouvé* | *51,41* | *5,93* | *8,79* | *3,56* |

### EXEMPLE 9 : Acide (2S,3aS,7aS)-1-[(2S)-2-{[(1S)-1-(éthoxycarbonyl)-butyl]-amino}-propionyl]-octahydro-1H-indole-2-carboxylique-Acide {2-[(3-{[5-oxydo-4-(phénylsulfonyl)-1,2,5-oxadiazol-3-yl]-oxy}-propionyl)-amino]-éthyl}-phosphonique (1:1)

### Stade A : Acide {[(3-{[5-oxydo-4-(phénylsulfony/)-1,2,5-oxadiazol-3-yl]-oxy}-propionyl)-amino]-éthyl}-phosphonique (composé B₇)

Le composé est obtenu selon le procédé décrit au Stade A de l'Exemple 7, en remplaçant l'acide aminométhylphosphonique par l'acide aminoéthylphosphonique.
*Point de fusion : 162°C.*

### Stade B : Acide (2S,3aS,7aS)-1-[(2S)-2-{[(1S)-1-(éthoxycarbonyl)-butyl]-amino}-propionyl]-octahydro-1H-indole-2-carboxylique-Acide {2-[(3-{[5-oxydo-4-(phénylsulfonyl)-1,2,5-oxadiazol-3yl]-oxy}-propionyl)-amino]-éthyl}-phosphonique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | 48,66 | 6,13 | *8,87* | *4,06* |
| *Trouvé* | *48,81* | *6, 06* | *8,90* | *4,42* |

### EXEMPLE 10 : Acide (2S,3aS,7aS)-1-[(2S)-2-{[(1S)-1-(éthoxycarbonyl)-butyl]-amino}-propionyl]-octahydro-1H-indole-2-carboxylique-Acide (2-{[5-oxydo-4-(phénylsulfonyl)-1,2,5-oxadiazol-3-yl]-oxy}-éthyl)-phosphonique (1:1)

### Stade A : (2-{[5-Oxydo-4-(phénylsulfonyl)-1,2,5-oxadiazol-3-yl]-oxy}-éthyl phosphonate de diméthyle

A une solution de 3,4-bis(phénylsulfonyl)-1,2,5-oxadiazole 2-oxyde (7,5 g - 20,7 mmol), et de 2-hydroxy-éthyl phosphonate de diméthyle (4,5 g - 31 mmol) dans 100 ml de THF, on additionne une solution de NaOH à 50% dans l'eau (1,5 g - 32 mmol) en 1/2h. Après 1h d'agitation on élimine le solvant sous vide et on reprend le résidu par de l'eau (100ml) et de l'acétate d'éthyle (100ml). La phase organique est séchée sur Na₂SO₄ puis évaporée à sec. On purifie le résidu par flash chromatographie en éluant par un mélange dichlorométhane/éthanol (95 /5) pour conduire au produit attendu sous la forme d'une huile incolore.

### Stade B : Acide (2-{[5-oxydo-4-(phénylsulfonyl)-1,2,5-oxadiazol-3-yl]-oxy}-éthyl)-phosphonique (composé B₁₀)

A une solution du composé obtenu au stade précédent (1,8 g - 4 mmol) dans 50 ml de dioxane, on additionne 2,9 ml de bromure de triméthylsilyle (4 eq.) puis chauffe le milieu réactionnel pendant 4 heures à 60°C.
Les solvants sont évaporés à sec. Le résidu est purifié sur une colonne Biogel éluée par un mélange eau / acétonitrile (1 /1). Les fractions contenant le produit pur sont lyophilisées.

### Stade C : Acide (2S,3aS,7aS)-1-[(2S)-2-{[(1S)-1-(éthoxycarbonyl)-butyl]-amino}-propionyl]-octahydro-1H-indole-2-carboxylique-Acide (2-{[5-oxydo-4-(phénylsulfonyl)-1,2,5-oxadiazol-3-yl]-oxy}-éthyl)-phosphonique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *48,46* | *6, 03* | *7,80* | *4,46* |
| *Trouvé* | *48,53* | *5,97* | *7,95* | *4,61* |

### EXEMPLE 12 : Acide (2S,3aS,7aS)-1-[(2S)-2-{[(1S)-1-(éthoxycarbonyl)-butyl]-amino}-propionyl]-octahydro-1H-indole-2-carboxylique-Acide N-(3-{[5-oxydo-4-(phénylsulfonyl-1,2,5-oxadiazol-3-yl]-oxy}-propionyl)-glycine (1:1)

### Stade A : N-(3-{[5-oxydo-4-(phénysulfonyl-1,2,5-oxadiazol-3-yl]-oxy}propionyl)-glycinate de terbutyle

Une solution du composé obtenu au stade B de l'Exemple 1 (1,5g-4 mmol), de glycinate de terbutyle (0,6g-4 mmol), EDCI (0,81g-4 mmol) et de HOBT (0,57g-4mmol) dans 100 ml de DMF anhydre est agitée 24h à température ambiante sous argon. Le DMF est alors éliminé par distillation et le résidu repris dans 100 ml d'eau et 100 ml d'acétate d'éthyle. La phase organique est lavée par une solution de NaHCO₃ à 5% dans l'eau, séchée sur Na₂SO₄ puis évaporée à sec. On purifie par flash chromatographie en éluant par un mélange dichlorométhane -acétate d'éthyle (95 :5) pour conduire au produit attendu sous la forme d'une huile incolore.

### Stade B : Acide N-(3-{[5-oxydo-4-(phénylsulfonyl-1,2,5-oxadiazol-3-yl]-oxy}propionyl)-glycine (composé B₁₃)

Une solution du composé obtenu au stade précédent (1,7g- 4mmol) dans 100 ml de dichlorométhane et 10 ml de TFA est agitée 6h à température ambiante.
Les solvants sont alors éliminés sous vide et le résidu trituré dans l'éther diéthylique pendant 2 heures jusqu'à cristallisation. Essorer, sécher sous vide de pompe.
*Point de fusion :* 177°C.

### Stade C : Acide (2S,3aS,7aS)-1-[(2S)-2-{[(1S)-1-(éthoxycarbonyl)-butyl]-amino}-propionyl]-octahydro-1H-indole-2-carboxylique-Acide N-(3-{[5-oxydo-4-(phénylsumonyl-1,2,5-oxadiazol-3-yl]-oxy}-propionyl)-glycine (1:1)

Le composé est obtenu selon le procédé décrit au Stade E de l'Exemple 1, à partir de perindopril et du composé obtenu au stade précédent.

### EXEMPLE 13 : Acide (2S)-2-[(4-{[5-oxydo-4-(phénylsulfonyl)-1,2,5-oxadiazol-3-yl]-oxy}-butanoyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide 4-[(5-oxydo-4-(phénylsulfonyl)-1,2,5-oxadiazol--3-yl)-oxy]-butanoïque (composé B₁₆)

Le composé attendu est obtenu selon le procédé décrit aux stades A et B de l'Exemple 1, en remplaçant au Stade A le 3-hydroxypropionate de terbutyle par le 4-hydroxybutanoate de terbutyle.
*Point de fusion :* 130-131°C

### Stade B : Acide (2S)-2-[(4-{[5-oxydo-4-(phénylsulfonyl)-1,2,5-oxadiazol-3-yl]-oxy}-butanoyl)-amino]-succinique (composé B₁₄).

Le composé attendu est obtenu selon le procédé décrit aux stades C et D de l'Exemple 1, à partir du composé obtenu au stade précédent.

### Stade C : Acide (2S)-2-[(4-{[5-oxydo-4-(phénylsulfonyl)-1,2,5-oxadiazol-3-yl]-oxy}-butanoyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | *51, 78* | *6, 08* | *8, 63* | *3, 95* |
| *Trouvé* | *52,52* | *6,05* | *8,82* | *3,47* |

### EXEMPLE 14 : Acide {[5-oxydo-4-(phénylsulfonyl)-1,2,5-oxadiazol-3-yl]-oxy}-acétique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### StadeA : Acide {[5-oxydo-4-(phénylsulfonyl)-1,2,5-oxadiazol-3-yl]oxy}acétique (composé B₁₅)

Le composé attendu est obtenu selon le procédé décrit aux stades A et B de l'Exemple 1, en remplaçant au Stade A le 3-hydroxypropionate de terbutyle par l'hydroxyacétate de terbutyle.

### Stade B : Acide {[5-oxydo-4-(phénylsulfonyl)-1,2,5-oxadiazol-3-yl]-oxy}-acétique - Acide (2S,3aS,7aS)-1-[(2S)-2-{[(1S)-1-(éthoxycarbonyl)-butyl]-amino}-propiony/]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | %C | *%H* | *%N* | %S |
| *Calculé* | *52, 09* | *6, 03* | *8, 38* | *4, 80* |
| *Trouvé* | *51,76* | *5,84* | *8,50* | *4,97* |

### EXEMPLE 15 : Acide 3-[(5-oxydo-4-(phénylsulfonyl)-1,2,5-oxadiazol-3-yl)-oxy]-propionique- Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade B de l'Exemple 1.

### EXEMPLE 16 : Acide 4-[(5-oxydo-4-(phénylsulfonyl)-1,2,5-oxadiazol--3-yl)-oxy]-butanoïque - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade A de l'Exemple 13.

### EXEMPLE 17 : Acide 3-({4-[(4-chlorophényl)sulfonyl]-5-oxydo-1,2,5-oxadiazol-3-yl}oxy)-propionique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide 3-({4-[(4-chlorophényl)sulfonyl]-5-oxydo-1,2,5-oxadiazol-3-yl}oxy)-propionique (composé B₁₇)

Le composé attendu est obtenu selon le procédé décrit aux stade A et B de l'Exemple 1 en remplaçant au stade A le 3,4-bis(phénylsulfonyl)-1,2,5-oxadiazole 2-oxyde par le 3,4-bis[(4-chlorophényl)-sulfonyl]-1,2,5-oxadiazole 2-oxyde.

### Stade B : Acide 3-({4-[(4-chlorophényl)sulfonyl]-5-oxydo-1,2,5-oxadiazol-3-yl}oxy)-propionique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

### EXEMPLE 18 : Acide (2S)-2-{[3-({4-[(4-chlorophényl)sulfonyl]-5-oxydo-1,2,5-oxadiazol-3-yl}oxy)propionyl]amino}succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-{[3-({4-[(4-chlorophényl)sulfonyl]-5-oxydo-1,2,5-oxadiazol-3-yl}oxy)propionyl]amino}succinique (composé B₁₈)

Le composé attendu est obtenu selon le procédé décrit aux stades C et D de l'Exemple 1 à partir du composé obtenu au stade A de l'Exemple 17.

### Stade B : Acide (2S)-2-{[3-({4-[(4-chlorophényl)sulfonyl]-5-oxydo-1,2,5-oxadiazol-3-yl}oxy)propionyl]amino}succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | | |
|---|---|---|---|---|---|
| | *% C* | *% H* | *% N* | *%S* | *%Cl* |
| *Calculé* | *49, 07* | *5,57* | *8,41* | *3,85* | *4, 26* |
| *Trouvé* | *49,12* | *5,82* | *8, 27* | *3,70* | *4,12* |

### EXEMPLE 19 : Acide (2S)-2-[(3-{[5-oxydo-4-(phénylsulfonyl)-1,2,5-oxadiazol-3-yl]-oxy}-acétyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-[(3-{[5-oxydo-4-(phénylsulfonyl)-1,2,5-oxadiazol-3-yl]-oxy}-acétyl)-amino]-succinique (composé B₂₄).

Le composé attendu est obtenu selon le procédé décrit aux stades A à D de l'Exemple 1 en remplaçant au stade A le 3-hydroxypropionate de terbutyle par l'hydroxyacétate de terbutyle.

### Stade B : Acide (2S)-2-[(3-{[5-oxydo-4-(phénylsulfonyl)-1,2,5-oxadiazol-3-yl]-oxy}-acétyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | 5*0,57* | *5,79* | *8,94* | *4,09* |
| *Trouvé* | *50,71* | *5,79* | *9, 04* | *3,82* |

### EXEMPLE 20 : Acide (2S)-2-[(2-{[5-oxydo-4-(phénylsulfonyl)-1,2,5-oxadiazol-3-yl]oxy}éthyl)amino]succinique : Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : (2S)-2-[(2-Hydroxyéthyl)amino]succinate de diterbutyle

Une solution de L-aspartate de diterbutyle (5,62 g - 20 mmol), de 2-bromoéthanol (2,84 mL - 40 mmol) et de K₂CO₃ (5,6 g - 40 mmol) dans 100 mL d'acétonitrile est portée à reflux pendant 16 heures. Les sels minéraux sont filtrés et le solvant éliminé par distillation sous vide. On purifie le produit brut ainsi obtenu par flash chromatographie sur gel de silice en utilisant comme éluant un mélange dichlorométhane - éthanol (95 :5). On obtient le produit attendu sous la forme d'une huile incolore.

### Stade B : Acide (2S)-2-[(2-{[5-oxydo-4-(phénylsulfonyl)-1,2,5-oxadiazol-3-yl]oxy}éthyl)amino]succinique (composé B₂₅)

Le composé est obtenu selon le procédé décrit aux stades A et B de l'Exemple 1, en remplaçant le 3-hydroxypropionate de terbutyle par le composé obtenu au stade précédent.
*Point de fusion : 160-161 °C.*

### Stade C : Acide (2S)-2-[(2-{[5-oxydo-4-(phénylsulfonyl)-1,2,5-oxadiazol-3-yl]oxy}éthyl)amino]succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *51,49* | *6,15* | *9,10* | *4,17* |
| *Trouvé* | *51,05* | *5,94* | *9,20* | *4,06* |

### EXEMPLE 21 : Acide (2S)-2-{[3-[(4-[(4-méthylphényl)sulfonyl]-5-oxydo-1,2,5-oxadiazol-3-yl}oxy)propionyl]amino}succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-{[3-({4-[(4-méthylphényl)sulfonyl]-5-oxydo-1,2,5-oxadiazol-3-yl}oxy)propionyl]amino}succinique (composé B₂₁)

Le composé est obtenu selon le procédé décrit aux Stades A à D de l'Exemple 1, en remplaçant au Stade A le 3,4-bis(phénylsulfonyl)-1,2,5-oxadiazole 2-oxyde par le 3,4-bis[(4-méthylphényl)sulfonyl]-1,2,5-oxadiazole 2-oxyde.
*Point de fusion : 167-168°C.*

### Stade B : Acide (2S)-2-{[3-({4-[(4-méthylphényl)sulfonyl]-5-oxydo-1,2,5-oxadiazol-3-yl}oxy)propionyl]amino}succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | *51,78* | *6,08* | *8,63* | *3,95* |
| *Trouvé* | *51,92* | *5,96* | *8,45* | *3,96* |

### EXEMPLE 22 : Acide (2S)-2-{[3-({4-[(4-chlorophényl)sulfonyl]-5-oxydo-1,2,5-oxadiazol-3-yl}oxy)butanoyl]amino}succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-{[3-({4-[(4-chlorophényl)sulfonyl]-5-oxydo-1,2,5-oxadiazol-3-yl}oxy)butanoyl]amino}succinique (composé B₂₆)

Le composé attendu est obtenu selon le procédé décrit aux stades A à D de l'Exemple 1, en remplaçant au Stade A le 3-hydroxypropionate de tert-butyle par le 4-hydroxybutanoate de tert-butyle, et le 3,4-bis(phénylsulfonyl)-1,2,5-oxadiazole 2-oxyde par le 3,4-bis[(4-chlorophényl)-sulfonyl]-1,2,5-oxadiazole 2-oxyde.
*Point de fusion : 145-146°C.*

### Stade B : Acide (2S)-2-{[3-({4-[(4-chlorophényl)sulfonyl]-5-oxydo-1,2,5-oxadiazol-3-yl}oxy)butanoyl]amino}succinique-Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | | |
|---|---|---|---|---|---|
| | *% C* | *%H* | *%N* | *%S* | *%Cl* |
| *Calculé* | *49, 67* | *5,72* | *8, 28* | *3,79* | *4,19* |
| *Trouvé* | *49,44* | *5,78* | *8,20* | *3,70* | *4,10* |

### EXEMPLE 23 : Acide (2S)-2-{[3-({4-[(4-méthylphényl)sulfonyl]-5-oxydo-1,2,5-oxadiazol-3-yl}oxy)butanoyl]amino}succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-{[3-({4-[(4-méthylphényl)sulfonyl]-5-oxydo-1,2,5-oxadiazol-3-yl}oxy)butanoyl]amino}succinique (composé B₂₇)

Le composé attendu est obtenu selon le procédé décrit aux stades A à D de l'Exemple 1, en remplaçant au Stade A le 3-hydroxypropionate de tert-butyle par le 4-hydroxybutanoate de tert-butyle, et le 3,4-bis(phénylsulfonyl)-1,2,5-oxadiazole 2-oxyde par le 3,4-bis[(4-méthylphényl)-sulfonyl]-1,2,5-oxadiazole 2-oxyde.
*Point de fusion : 147-148°C.*

### Stade B : Acide (2S)-2-{[3-({4-[(4-méthylphényl)sulfonyl]-5-oxydo-1,2,5-oxadiazol-3-yl}oxy)butanoyl]amino}succinique - Acide (2S,3aS,7aS)-1-[(2S)-2-{[(1S)-1-(éthoxycarbonyl)-butyl]-amino}-propionyl)-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *52,36* | *6,22* | *8,48* | *3,88* |
| *Trouvé* | *52, 66* | *6,15* | *8,67* | *4,16* |

### EXEMPLE 24 : Acide 2-[(3-{[5-oxydo-4-(phénylsulfonyl)-1,2,5-oxadiazol-3-yl]-oxy}-butanoyl)-amino]-éthanesulfonique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide 2-[(3-{[5-oxydo-4-(phénylsulfonyl)-1,2,5-oxadiazol-3-yl]-oxy}-butanoyl)-amino]-éthanesulfonique (composé B₂₈)

Le composé attendu est obtenu selon le procédé décrit aux Stades A et B de l'Exemple 6, en remplaçant au Stade A le composé B₄ par le composé B₁₆ obtenu au Stade A de l'Exemple 13.
*Point de fusion : 131-132°C.*

### Stade B : Acide 2-[(3-{[5-oxydo-4-(phénylsufonyl)-1,2,5-oxadiazol-3-yl]-oxy}-butanoyl)-amino]-éthanesulfonique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *49,30* | *6,14* | *8,71* | *7,98* |
| *Trouvé* | *49,15* | *6,12* | *8,82* | *8,22* |

### EXEMPLE 25 : N,N-Bis(2-sulfoéthyl)-4-{[5-oxydo-4-(phénylsulfonyl)-1,2,5-oxadiazol-3-yl]oxy}butanamide - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : N-{2-[(3-{[5-oxydo-4-(phénylsulfonyl)-1,2,5-oxadiazol-3-yl]-oxy}-butanoyl)-oxy]}-5-norbornène-2,3-dicarboximide

Le composé attendu est obtenu selon le procédé décrit au Stade A de l'Exemple 6, en remplaçant le composé B₄ par le composé B₁₆ obtenu au Stade A de l'Exemple 13.

### Stade B : N,N-Bis(2-sulfoéthyl)-4-{[5-oxydo-4-(phénylsulfonyl)-1,2,5-oxadiazol-3-yl]oxy}butanamide (composé B₂₉)

Le composé attendu est obtenu selon le procédé décrit au Stade B de l'Exemple 6, à partir de ditaurine (préparée selon DE 10033580) et du composé obtenu au stade précédent.

### Stade C : N,N-Bis(2-sulfoéthyl)-4-{[5-oxydo-4-(phénylsulfonyl)-1,2,5-oxadiazol-3-yl]oxy}butanamide - Acide (2S,3aS,7aS)-1-[(2S)-2-{[(1S)-1-(éthoxycarbonyl)-buty/]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

### ETUDE PHARMACOLOGIQUE DES PRODUITS DE L'INVENTION

### EXEMPLE 26 : Activité donneur de NO

### In vitro

Les anneaux d'aorte sans endothélium sont utilisés. Après une première contraction induite par du KC1 60 mM pour caractériser la sensibilité de l'anneau et un lavage, une contraction stable est induite par de la noradrenaline (0,1-0,3 µM) en présence ou non de l'inhibiteur de la guanylate cyclase, l'ODQ (10 µM). Une gamme de concentrations cumulées est réalisée et l'activité du produit à tester est calculée par une IC₅₀ (dose inhibant de 50 % l'effet maximal).
Résultats : les composés selon l'invention présentent un effet relaxant tout à fait significatif ;
à titre d'exemple, les composés des Exemples 1 et 7 ont des IC₅₀ de 0,048 et 0,047 µM respectivement.

### EXEMPLE 27 : Activité inhibitrice de l'enzyme de conversion de l'angiotensine I

### In vivo

Les rats sont anesthésiés au pentobarbital, placés en respiration artificielle, la pression artérielle moyenne est mesurée par un cathéter placé dans l'artère fémorale et relié à un capteur de pression. Les nerfs vagues sont sectionnés et la mécamilamine (bloqueur des ganglions sympathiques) est injectée i.v. à 1.5 mg/kg. Après stabilisation, l'angiotensine I est injectée i.v. à 1.5 µg/kg.
La réponse hypertensive maximale à l'angiotensine I est mesurée sur des rats ayant reçu 60 minutes avant l'anesthésie, par voie orale soit de la gomme du Sénégal seule (groupe contrôle), soit la molécule testée dans la gomme du Sénégal. L'effet inhibiteur est déterminé en % de la réponse à l'angiotensine I versus le groupe contrôle.
Résultats : les composés selon l'invention présentent un effet inhibiteur tout à fait significatif ;
à titre d'exemple, les composés des Exemples 1 et 7, injectées à la dose de 0,1 mg/kg provoquent des inhibitions de l'angiotensine I de 85 et 88 % respectivement.

### EXEMPLE 28 : Composition pharmaceutique

| Formule de préparation pour 1000 comprimés dosés à 100 mg | |
|---|---|
| Composé de l'un des Exemples 1, 3, 4, 6 à 10 ou 12 à 25 | 100 g |
| Hydroxypropycellulose | 2 g |
| Amidon de blé | 10 g |
| Lactose | 100 g |
| Stéarate de magnésium | 3 g |
| Talc | 3 g |

## Revendications

1. Composés de formule (I) :
A • B (I)
dans laquelle A représente le perindopril ou le perindoprilate, et
B est un composé comportant au moins une fonction acide salifiable représenté par la formule (III) suivante :
X-(Ak₁)ₓ-(Y)_{y}-(Ak₂)_{z}-Z (III)
dans laquelle :
- X représente un groupement CO₂H, SO₃H ou P(O)(OH)₂,
- Ak₁ et Ak₂, identiques ou différents, représentent chacun un groupement alkylène C₁-C₈ linéaire ou ramifié, saturé ou insaturé, dans lequel un ou plusieurs des atomes de carbone peuvent être remplacés par un atome d'oxygène, de soufre, d'azote ou un groupement SO₂, ledit groupement alkylène étant éventuellement substitué par un ou plusieurs groupements choisis parmi CO₂H, SO₃H, hydroxy et amino,
- x, y et z, identiques ou différents, représentent chacun 0 ou 1,
- Y représente CO ou CONH,
- Z représente un groupement donneur de NO choisi parmi les groupements suivants 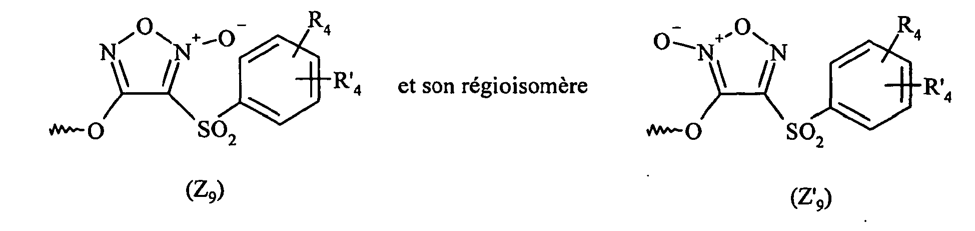 où R₄ et R'₄, identiques ou différents, représentent chacun un atome d'hydrogène ou d'halogène ou un groupement alkyle C₁-C₆ linéaire ou ramifié, trifluorométhyle ou trifluorométhoxy,
étant entendu que la liaison entre A et B est de type ionique.

2. Composés de formule (I) selon la revendication 1, où le composé de formule (III) est choisi parmi les composés de formules suivantes :
HO₂C-(Ak₁)ₓ-Z (IIIa)
HO₂C-CH=CH-(Ak₃)ₓ-Z (IIIb) 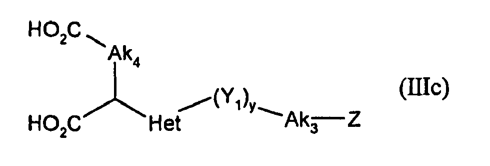 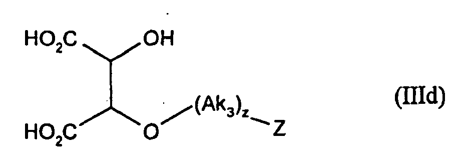 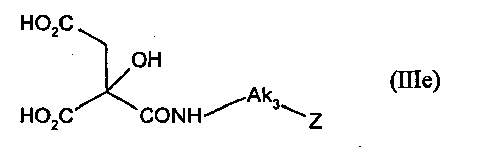 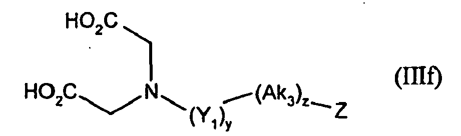 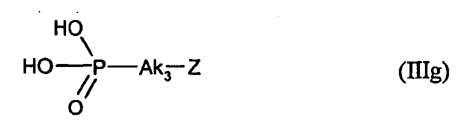 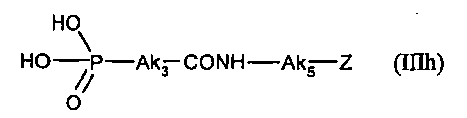
HO₃S-Ak₆-CONH-Ak₅-Z (IIIi) 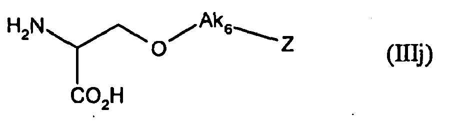 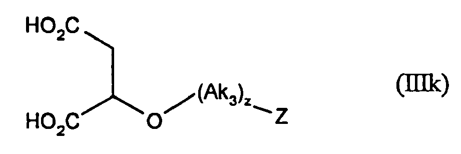
dans lesquelles :
Ak₁, x, y, z et Z sont tels que définis dans la revendication 1,
Ak₃ et Ak₅, identiques ou différents, représentent chacun un groupement alkylène C₁-C₆ linéaire ou ramifié saturé dans lequel un ou plusieurs des atomes de carbone peuvent être remplacés par un atome d'oxygène, de soufre, d'azote ou un groupement SO₂,
Ak₄ représente un groupement alkylène C₁-C₃ linéaire ou ramifié saturé dans lequel un des atomes de carbone peut être remplacé par un atome d'oxygène, de soufre, d'azote ou un groupement SO₂,
Ak₆ représente un groupement alkylène C₂-C₅ linéaire ou ramifié saturé dans lequel un des atomes de carbone peut être remplacé par un atome d'oxygène, de soufre, d'azote ou un groupement SO₂,
Het représente O ou NH,
et Y₁ représente un groupement carbonyle.

3. Composés de formule (I) selon l'une quelconque des revendications 1 ou 2, dans laquelle B est choisi parmi les composés suivants :

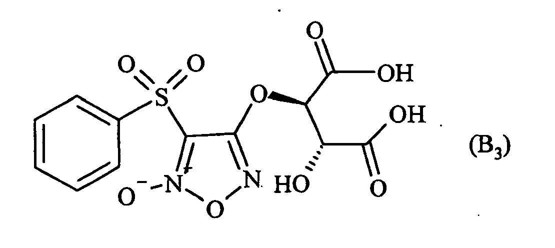

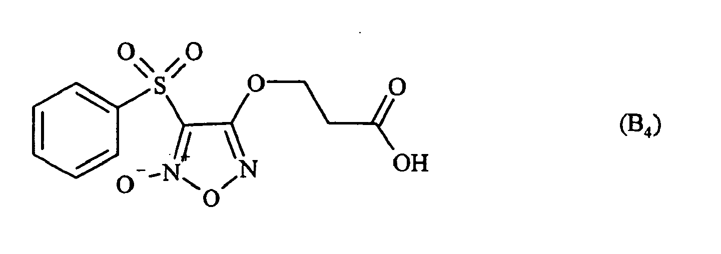

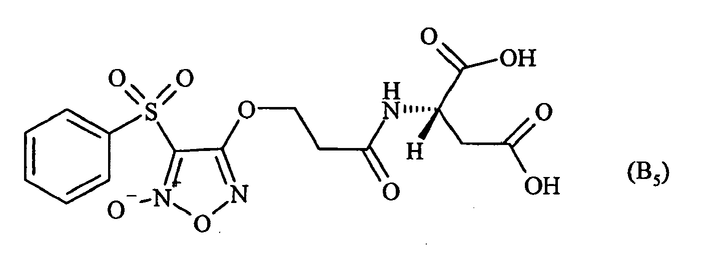

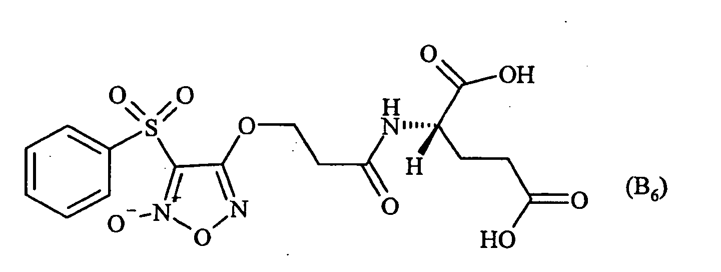

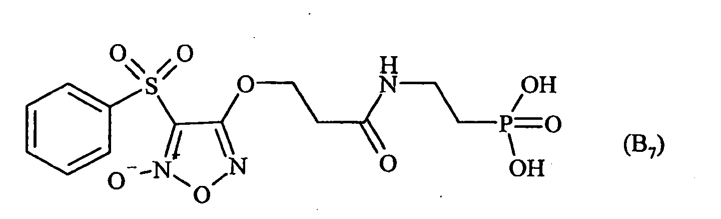

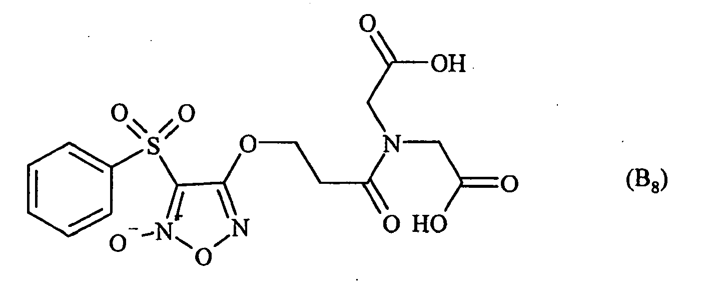

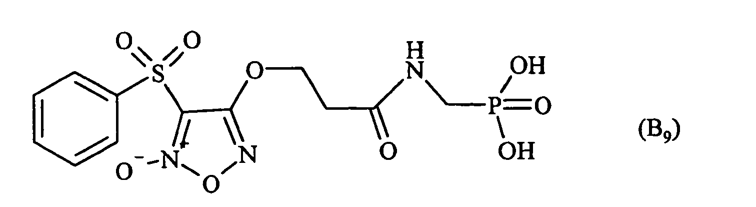

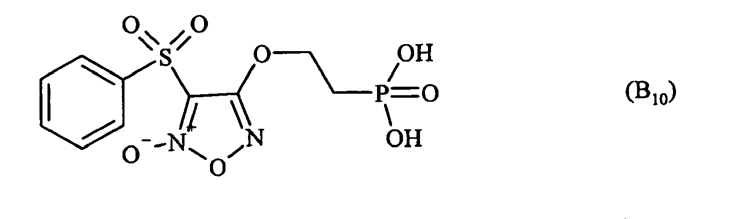

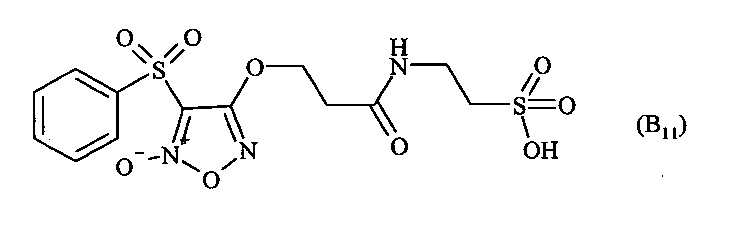

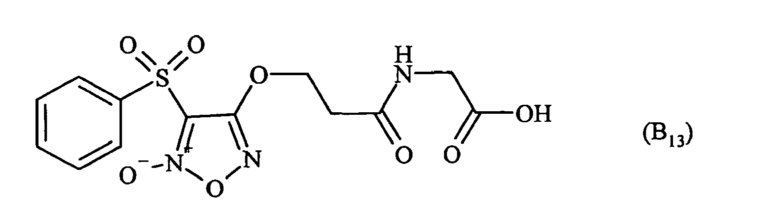

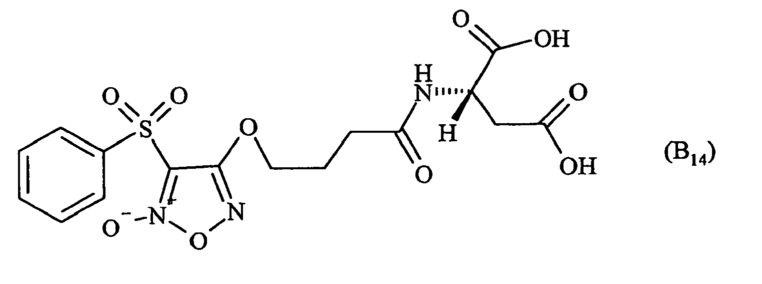

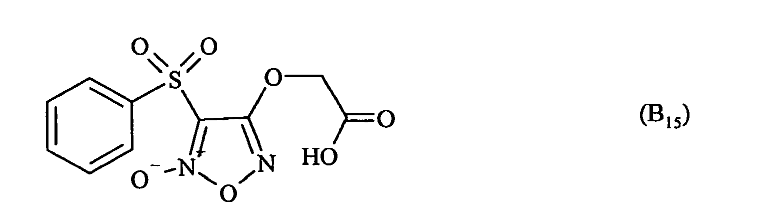

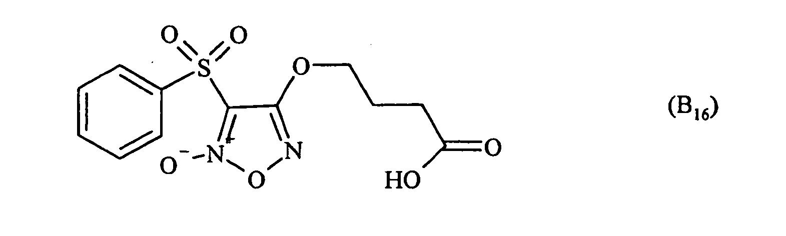

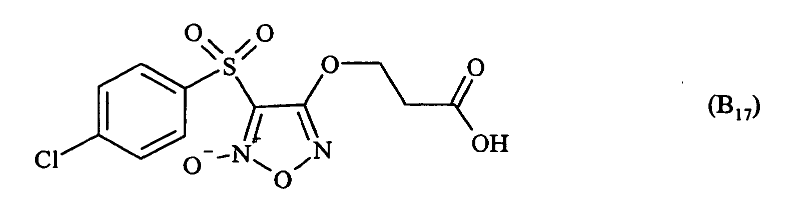

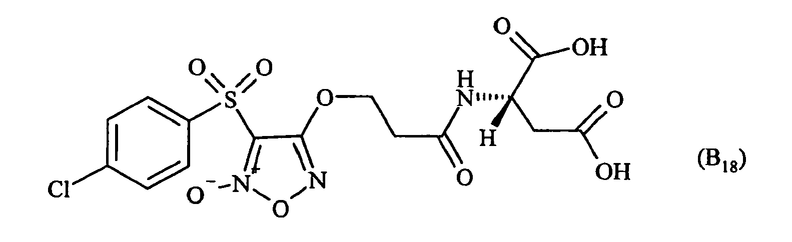

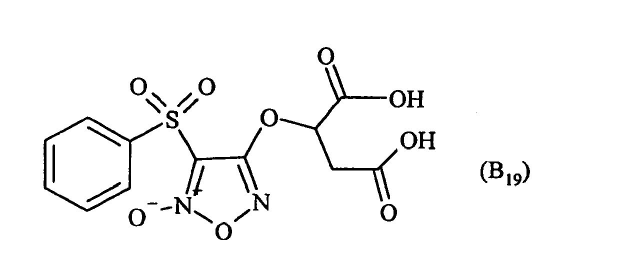

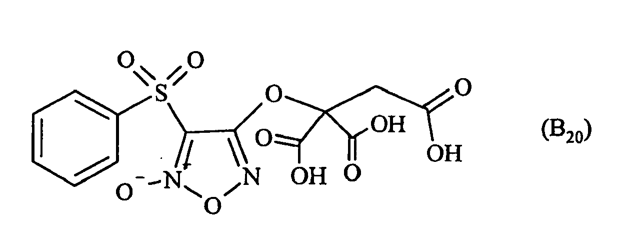

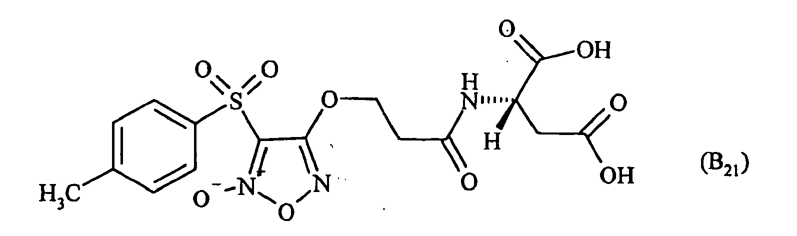

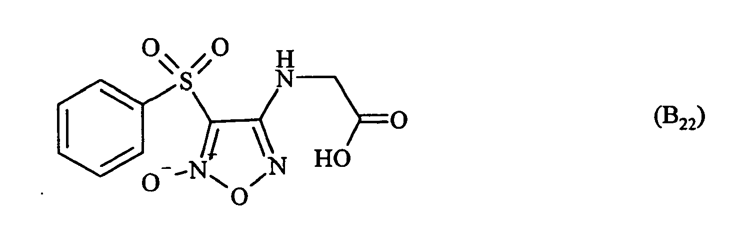

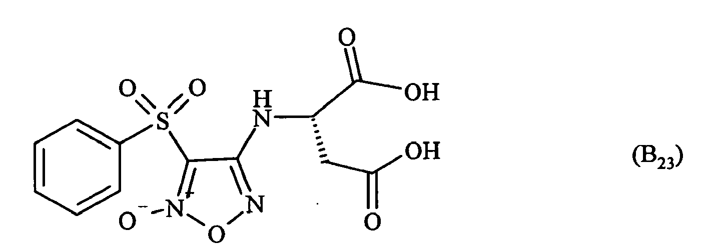

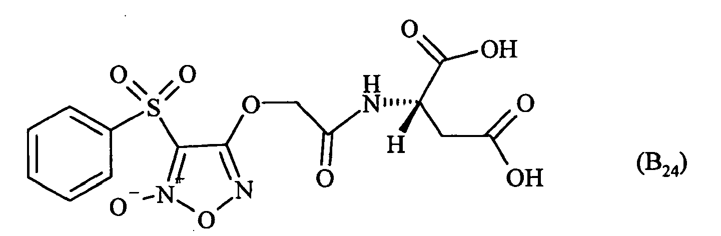

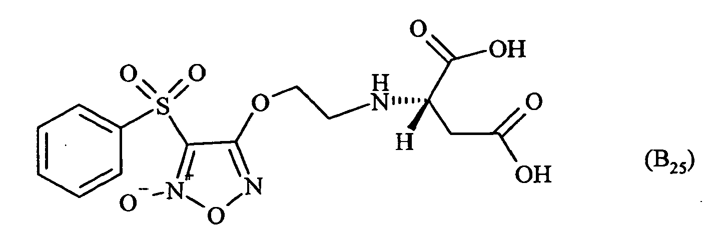

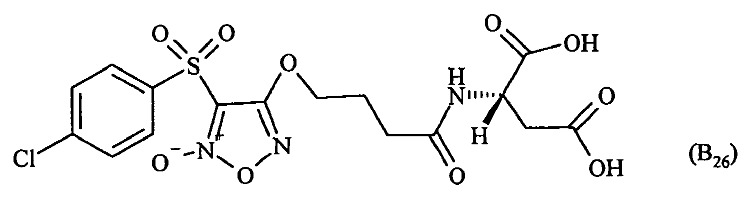

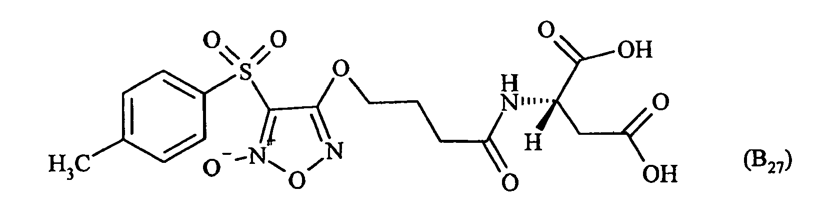

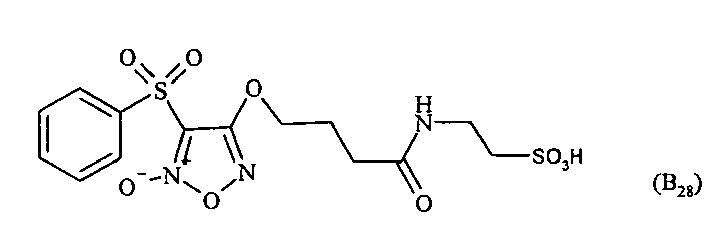

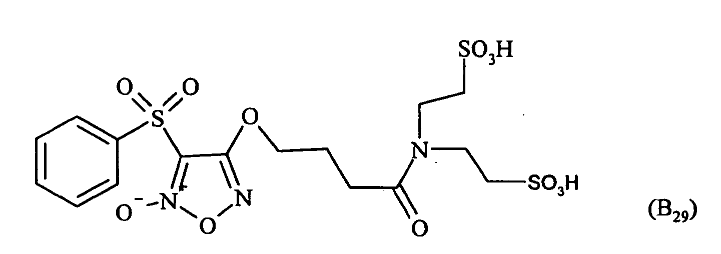

4. Composés de formule (I) selon la revendication 1, choisis parmi les composés suivants:
- Acide (2*S*)-2-[(3-{[5-oxydo-4-(phénylsulfonyl)-1,2,5-oxadiazol-3-yl]-oxy}-propionyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1);
- Acide (2*S*)-2-[(3-{[5-oxydo-4-(phénylsulfonyl)-1,2,5-oxadiazol-3-yl]-oxy}-propionyl)-amino]-pentanedioique - Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique (1:1);
- Acide 2-[(3-{[5-oxydo-4-(phénylsulfonyl)-1,2,5-oxadiazol-3-yl]-oxy}-propionyl)-amino]-éthanesulfonique - Acide (2*S*,3a*S*,7a*S*)-1-[(2*S*)-2-{[(1*S*)-1-(éthoxycarbonyl)-butyl]-amino}-propionyl]-octahydro-1*H-*indole-2-carboxylique (1:1);
- Acide (2*S*,3a*S*,7a*S*)-1-[(2*S*)-2-{[(1*S*)-1-(éthoxycarbonyl)-butyl]-amino}-propionyl]-octahydro-1*H-*indole-2-carboxylique - Acide {[(3-{[5-oxydo-4-(phénylsulfonyl)-1,2,5-oxadiazol-3-yl]-oxy}-propionyl)-amino]-méthyl}-phosphonique (1:1);
- Acide (2*S*,3a*S*,7a*S*)-1-[(2*S*)-2-{[(1*S*)-1-(éthoxycarbonyl)-butyl]-amino}-propionyl]-octahydro-1*H-*indole-2-carboxylique - Acide 2,2'-[(3-{[5-oxydo-4-(phénylsulfonyl)-1,2,5-oxadiazol-3-yl]-oxy}-propionyl)-imino]-diacétique (1:1);
- Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique-Acide {2-[(3-{[5-oxydo-4-(phénylsulfonyl)-1,2,5-oxadiazol-3-yl]-oxy}-propionyl)-amino]-éthyl}-phosphonique (1:1);
- Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique - Acide (2-{[5-oxydo-4-(phénylsulfonyl)-1,2,5-oxadiazol-3-yl]-oxy}-éthyl)-phosphonique (1:1);
- Acide (2S)-2-[(4-{[5-oxydo-4-(phénylsulfonyl)-1,2,5-oxadiazol-3-yl]-oxy}-butanoyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1);
- Acide (2S)-2-{[3-({4-[(4-chlorophényl)sulfonyl]-5-oxydo-1,2,5-oxadiazol-3-yl}oxy)propionyl]amino}succinique- Acide (2S,3aS,7aS)-1-[(2S)-2-{[(1S)-1-(éthoxycarbonyl)-butyl]-amino}-propionyl]-octahydro-1H-indole-2-carboxylique (1:1);
- Acide (2S)-2-{[3-({4-[(4-méthylphényl)sulfonyl]-5-oxydo-1,2,5-oxadiazol-3-yl}oxy)propionyl]amino}succinique - Acide (2S,3aS,7aS)-1-[(2S)-2-{[(1*S*)-1-(éthoxycarbonyl)-butyl]-amino}-propionyl]-octahydro-1*H*-indole-2-carboxylique (1:1);
- Acide (2S)-2-{[3-({4-[(4-méthylphényl)sulfonyl]-5-oxydo-1,2,5-oxadiazol-3-yl}oxy)butanoyl]amino}succinique - Acide (2S,3aS,7aS)-1-[(2S)-2-{[(1*S*)-1-(éthoxycarbonyl)-butyl]-amino}-propionyl]-octahydro-1*H-*indole-2-carboxylique (1:1).

5. Composés de formule (III) :
X-(Ak₁)ₓ-(Y)_{y}-(Ak₂)_{z}-Z (III)
dans laquelle :
- X représente un groupement CO₂H, SO₃H ou P(O)(OH)₂,
- Ak₁ et Ak₂, identiques ou différents, représentent chacun un groupement alkylène C₁-C₈ linéaire ou ramifié, saturé ou insaturé, dans lequel un ou plusieurs des atomes de carbone peuvent être remplacés par un atome d'oxygène, de soufre, d'azote ou un groupement SO₂, ledit groupement alkylène étant éventuellement substitué par un ou plusieurs groupements choisis parmi carboxy, SO₃H, hydroxy et amino,
- x, y et z, identiques ou différents, représentent chacun 0 ou 1,
- Y représente CO ou CONH,
- Z représente un groupement Z₉ ou Z'₉ : 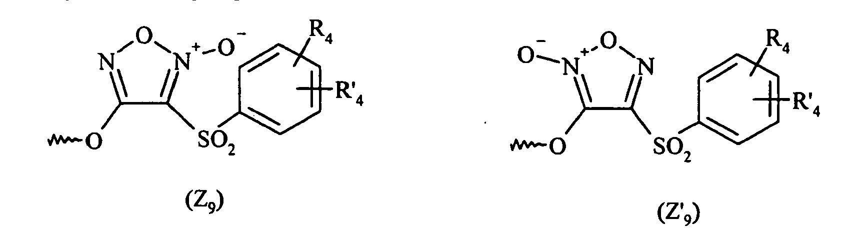 où R₄ et R'₄, identiques ou différents, représentent chacun un atome d'hydrogène ou d'halogène ou un groupement alkyle C₁-C₆ linéaire ou ramifié, trifluorométhyle ou trifluorométhoxy.

6. Procédé de synthèse des composés de formule (I) selon la revendication 1, dans lequel le composé A est mis en réaction avec le composé B en quantité au moins égale à 1 équivalent du composé A.

7. Composition pharmaceutique contenant comme principe actif un composé de formule (I) selon l'une quelconque des revendications 1 à 5, en combinaison avec un ou plusieurs excipients ou véhicules inertes, non toxiques, pharmaceutiquement acceptables.

8. Composition pharmaceutique selon la revendication 7 utile pour le traitement des pathologies cardiovasculaires.

9. Composition pharmaceutique selon la revendication 8 utile pour le traitement de l'hypertension artérielle et ses conséquences vasculaires et rénales, l'hypertension systolique, les maladies vasculaires périphériques, l'athérosclérose, la resténose, l'insuffisance cardiaque, la thrombose et tous les événements thromboemboliques, l'angine de poitrine stable ou instable, les accidents vasculaires cérébraux, les accidents coronariens, l'infarctus du myocarde, le remodelage vasculaire, le diabète et ses conséquences vasculaires et rénales, les complications liées aux interventions chirurgicales cardiovasculaires ou la dysfonction endothéliale.

10. Composition pharmaceutique selon la revendication 7 utile pour le traitement des pathologies impliquant l'inflammation et le stress oxydant.

## Claims

1. Compounds of formula (I):
A • B (I)
wherein A represents perindopril or perindoprilate, and
B is a compound containing at least one salt-forming acid function represented by the following formula (III):
X-(Ak₁)ₓ-(Y)_{y}-(Ak₂)_{z}-Z (III)
wherein:
- X represents a CO₂H, SO₃H or P(O)(OH)₂ group,
- Ak₁ and Ak₂, which may be identical or different, each represents a saturated or unsaturated, linear or branched C₁-C₈alkylene group in which one or more of the carbon atoms may be replaced by an oxygen, sulphur or nitrogen atom or by an S_{O}2 group, the said alkylene group optionally being substituted by one or more groups selected from CO₂H, SO₃H, hydroxy and amino,
- x, y and z, which may be identical or different, each represents 0 or 1,
- Y represents CO or CONH,
- Z represents an NO donor group selected from the following groups: 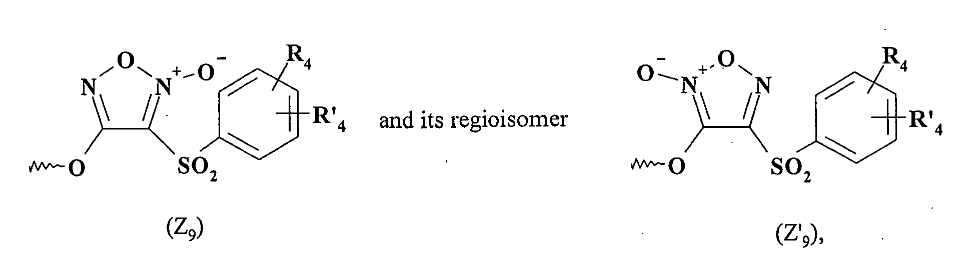 wherein R₄ and R'₄, which may be identical or different, each represents a hydrogen or halogen atom or a linear or branched C₁-C₆alkyl, trifluoromethyl or trifluoromethoxy group,
it being understood that the bond between A and B is of the ionic type.

2. Compounds of formula (I) according to claim 1, wherein the compound of formula (III) is selected from compounds of the following formulae:
HO₂C-(Ak₁)ₓ-Z (IIIa)
HO₂C-CH=CH-(Ak₃)ₓ-Z (IIIb) 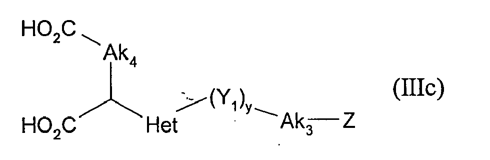 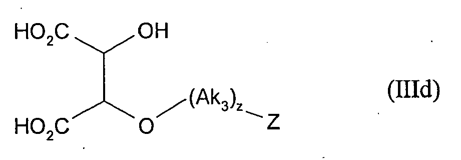 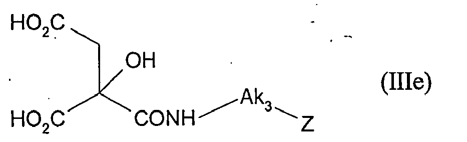 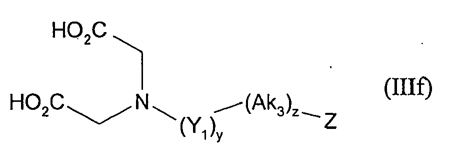 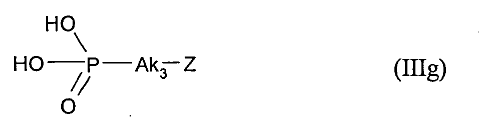 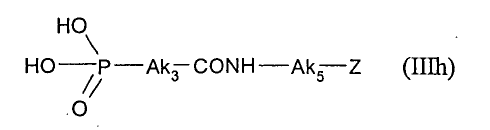
HO₃S-Ak₆-CONH-Ak₅-Z (IIIi) 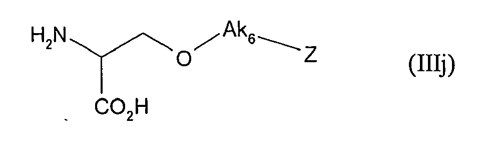 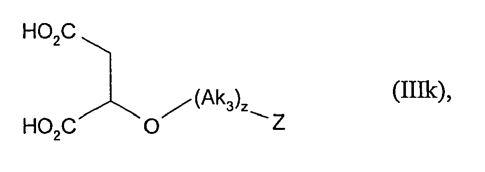
in which formulae:
Ak₁, x, y, z and Z are as defined in claim 1,
Ak₃ and Ak₅, which may be identical or different, each represents a saturated linear or branched C₁-C₆alkylene group in which one or more of the carbon atoms may be replaced by an oxygen, sulphur or nitrogen atom or by an SO₂ group,
Ak₄ represents a saturated linear or branched C₁-C₃alkylene group in which one of the carbon atoms may be replaced by an oxygen, sulphur or nitrogen atom or by an SO₂ group,
Ak₆ represents a saturated linear or branched C₂-C₅alkylene group in which one of the carbon atoms may be replaced by an oxygen, sulphur or nitrogen atom or by an SO₂ group,
Het represents O or NH,
and Y₁ represents a carbonyl group.

3. Compounds of formula (I) according to either claim 1 or claim 2, wherein B is selected from the following compounds:

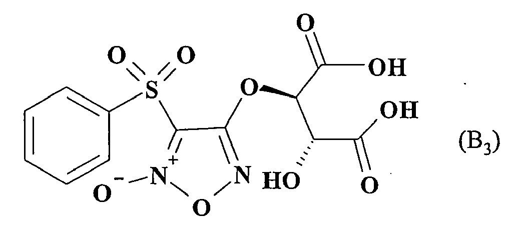

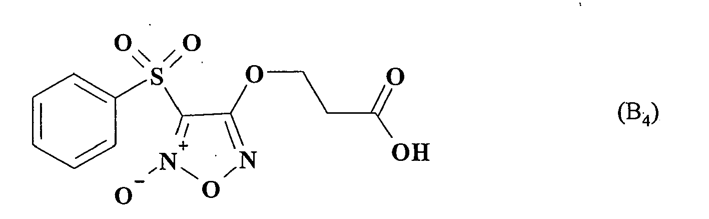

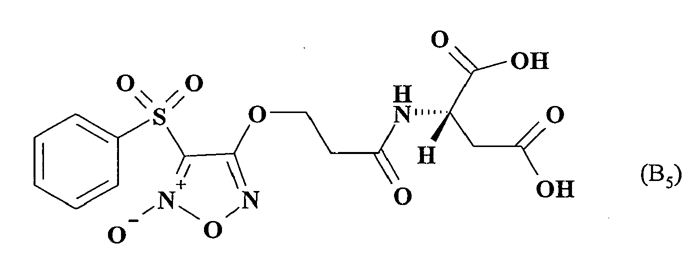

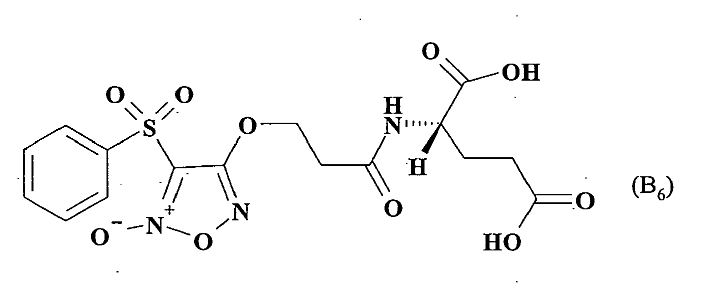

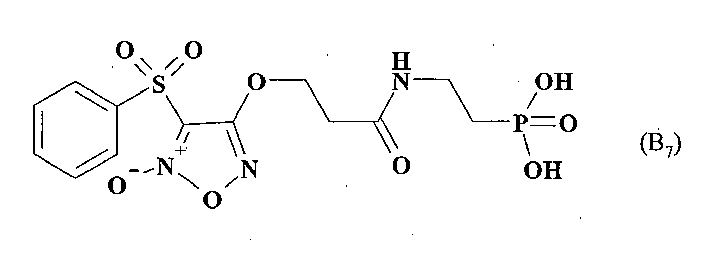

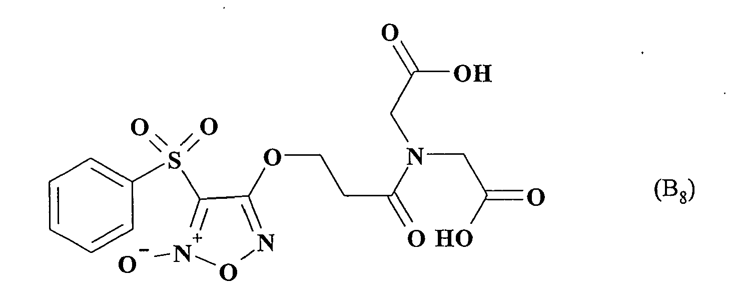

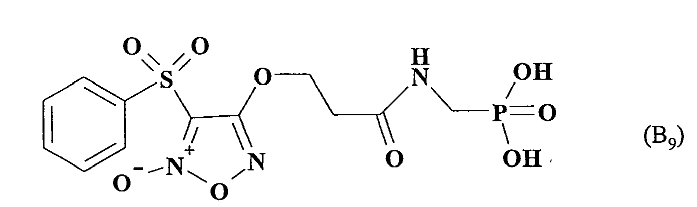

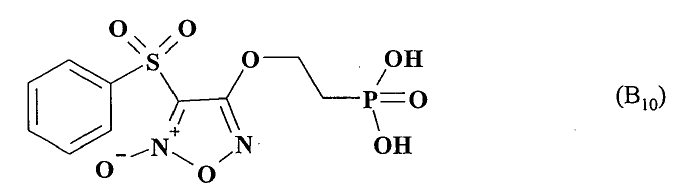

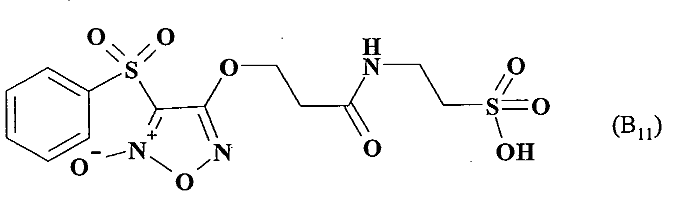

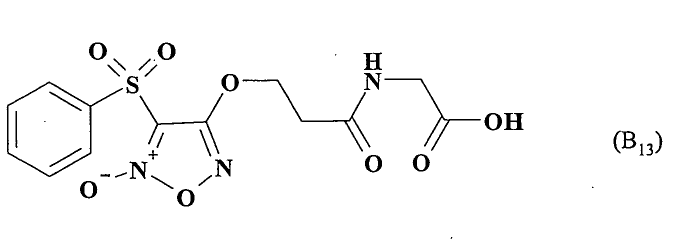

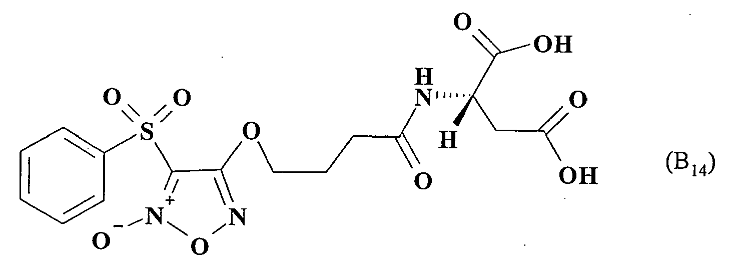

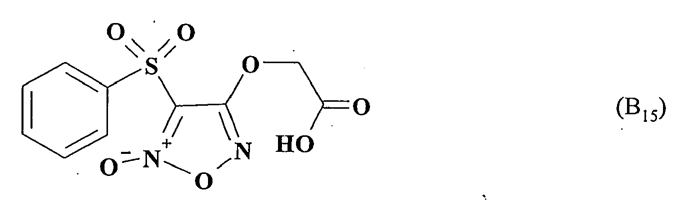

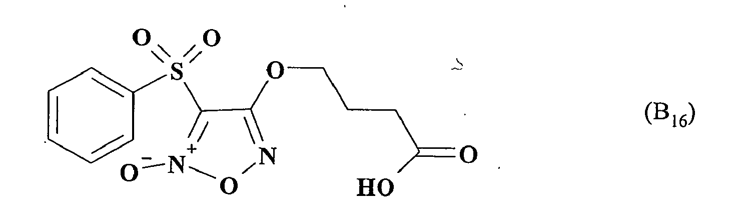

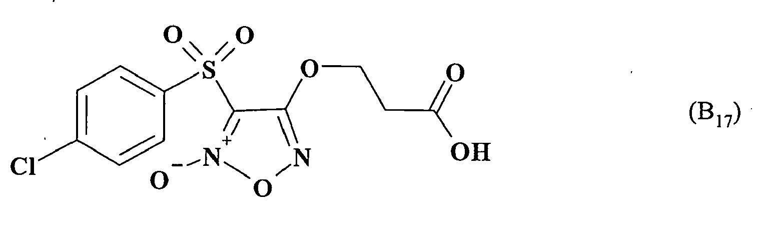

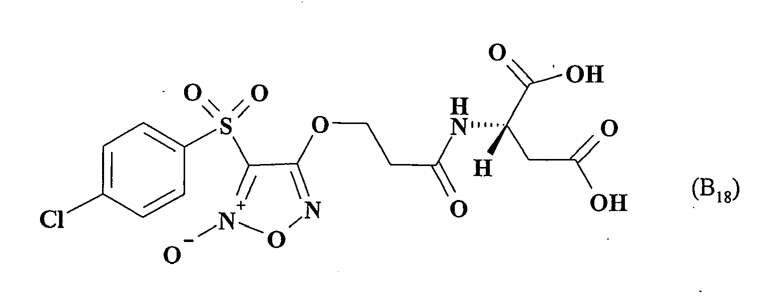

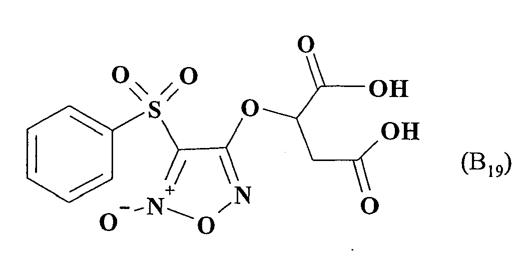

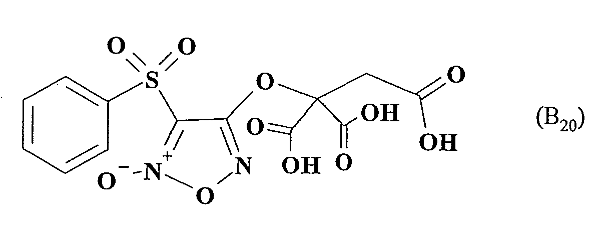

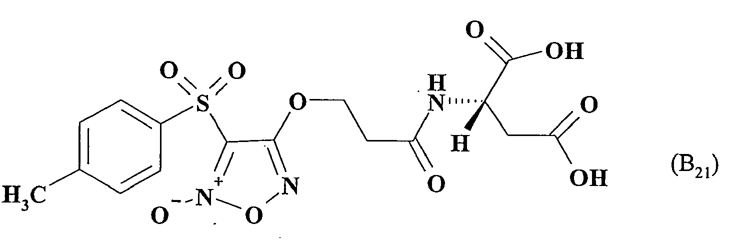

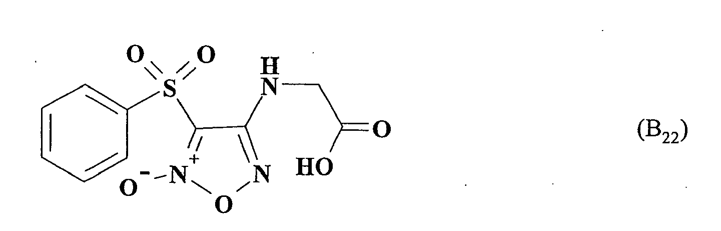

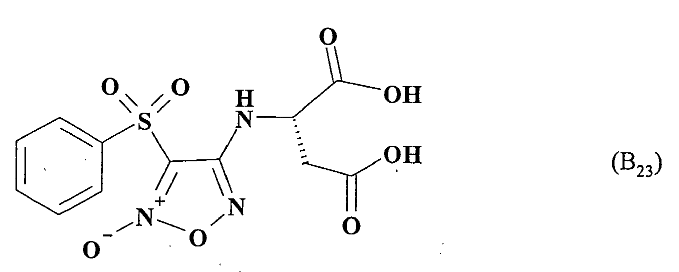

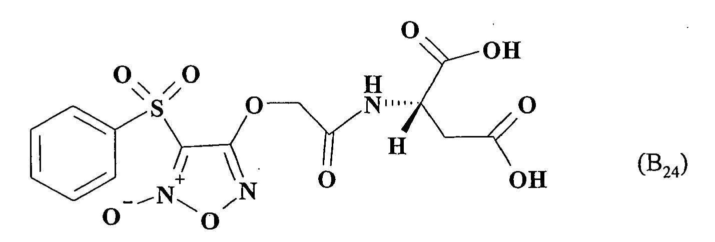

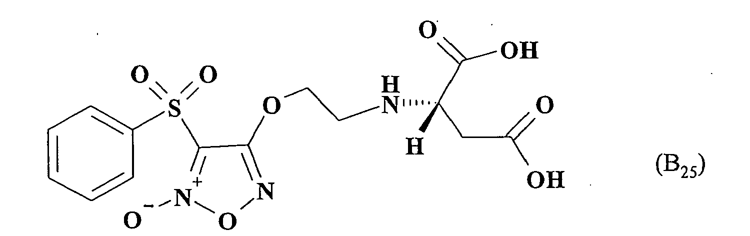

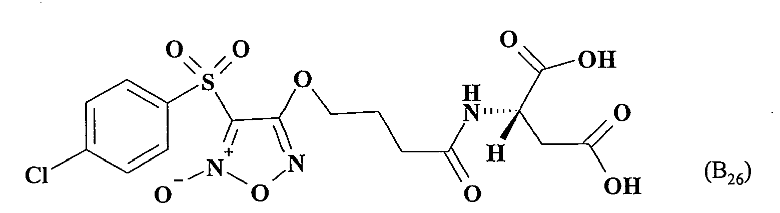

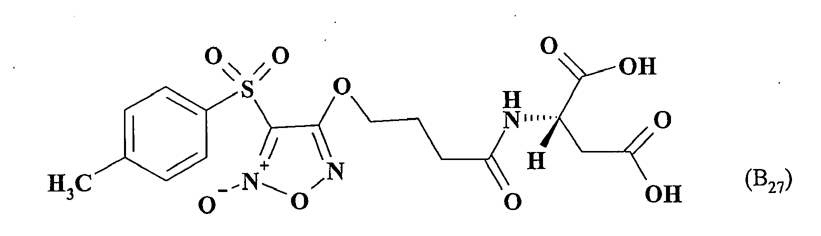

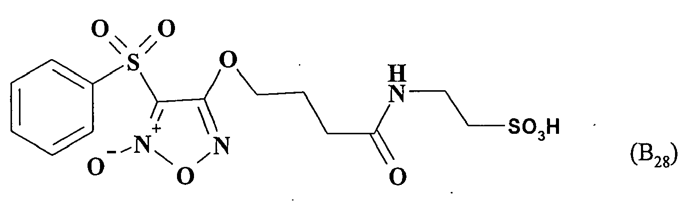

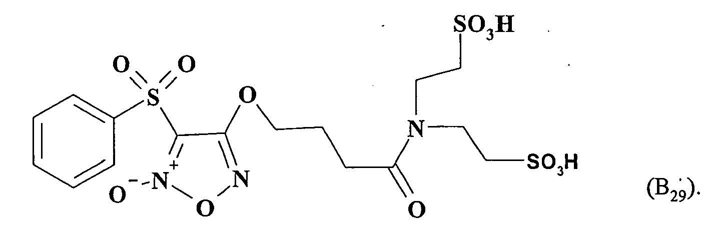

4. Compounds of formula (I) according to claim 1, selected from the following compounds:
- (2*S*)-2-[(3-{[5-oxido-4-(phenylsulphonyl)-1,2,5-oxadiazol-3-yl]-oxy}-propionyl)-amino]-succinic acid - (25,3aS,7aS)-1-[(25)-2-{[1S)-1-(ethoxycarbonyl)-butyl]-amino}-propionyl]-octahydro-1H-indole-2-carboxylic acid (1:1);
- (2*S*)-2-[(3-{[5-oxido-4-(phenylsulphonyl)-1,2,5-oxadiazol-3-yl]-oxy}-propionyl)-amino]-pentanedioic acid - (2*S*,3a*S*,7a*S*)-1-[(2*S*)-2-{[(1*S*)-1-(ethoxycarbonyl)-butyl]-amino}-propionyl]-octahydro-1*H-*indole-2-carboxylic acid (1:1);
- 2-[(3-{[5-oxido-4-(phenylsulphonyl)-1,2,5-oxadiazol-3-yl]-oxy}-propionyl)-amino]-ethanesulphonic acid - (2*S*,3a*S*,7a*S*-1-[(2*S*)-2-{[(1*S*-1-(ethoxycarbonyl)-butyl]-amino}-propionyl]-octahydro-1*H-*indole-2-carboxylic acid (1:1);
- (2*S*,3a*S*,7a*S*)-1-[(2S)-2-{[(1*S*)-1-(ethoxycarbonyl)-butyl]-amino}-propionyl]-octahydro-1*H-*indole-2-carboxylic acid - {[(3-{[5-oxido-4-(phenylsulphonyl)-1,2,5-oxadiazol-3-yl]-oxy}-propionyl)-amino]-methyl}-phosphonic acid (1:1);
- (2*S*,3a*S*,7a*S*)-1-[(2*S*)-2-{[(1*S*)-1-(ethoxycarbonyl)-butyl]-amino}-propionyl]-octahydro-1*H-*indole-2-carboxylic acid - 2,2'-[(3-{[5-oxido-4-(phenylsulphonyl)-1,2,5-oxadiazol-3-yl]-oxy}-propionyl)-imino]-diacetic acid (1:1);
- (2*S*,3a*S*,7a*S*)-1-[(2*S*)-2-{[(1*S*)-1-(ethoxycarbonyl)-butyl]-amino}-propionyl]-octahydro-1*H-*indole-2-carboxylic acid - {2-[(3-{[5-oxido-4-(phenylsulphonyl)-1,2,5-oxadiazol-3-yl]-oxy}-propionyl)-amino]-ethyl}-phosphonic acid (1:1);
- (2*S*,3a*S*,7a*S*)-1-[(2*S*)-2-{[(1*S*)-1-(ethoxycarbonyl)-butyl]-amino}-propionyl]-octahydro-1*H*-indole-2-carboxylic acid - (2-{[5-oxido-4-(phenylsulphonyl)-1,2,5-oxadiazol-3-yl]-oxy}-ethyl)-phosphonic acid (1:1);
- (2S)-2-[(4-{[5-oxido-4-(phenylsulphonyl)-1,2,5-oxadiazol-3-yl]-oxy}-butanoyl)-amino]-succinic acid - (2S,3aS,7aS)-1-[(2S)-2-{[(1S)-1-(ethoxycarbonyl)-butyl]-amino}-propionyl]-octahydro-1H-indole-2-carboxylic acid (1:1);
- (2S)-2-{[3-({4-[(4-chlorophenyl)sulphonyl]-5-oxido-1,2,5-oxadiazol-3-yl}oxy)-propionyl]amino}succinic acid - (2S,3aS,7aS)-1-[(2S)-2-{[(1S)-1-(ethoxycarbonyl)-butyl]-amino}-propionyl]-octahydro-1H-indole-2-carboxylic acid (1:1);
- (2S)-2-{[3-({4-[(4-methylphenyl)sulphonyl]-5-oxido-1,2,5-oxadiazol-3-yl}oxy)-propionyl]amino}succinic acid - (2S,3aS,7aS)-1-[(2S)-2-{[(1S)-1-(ethoxycarbonyl)-butyl]-amino}-propionyl]-octahydro-1*H*-indole-2-carboxylic acid (1:1);
- (2S)-2-{[3-({4-[(4-methylphenyl)sulphonyl]-5-oxido-1,2,5-oxadiazol-3-yl}oxy)-butanoyl]amino}succinic acid - (2S,3aS,7aS)-1-[(2S)-2-{[(1S)-1-(ethoxycarbonyl)-butyl]-amino}-propionyl]-octahydro-1*H*-indole-2-carboxylic acid (1:1).

5. Compounds of formula (III):
X-(Ak₁)ₓ-(Y)_{y}-(Ak₂)_{z}-Z (III)
wherein:
- X represents a CO₂H, SO₃H or P(O)(OH)₂ group,
- Ak₁ and Ak₂, which may be identical or different, each represents a saturated or unsaturated, linear or branched C₁-C₈alkylene group in which one or more of the carbon atoms may be replaced by an oxygen, sulphur or nitrogen atom or by an SO₂ group, the said alkylene group optionally being substituted by one or more groups selected from carboxy, SO₃H, hydroxy and amino,
- x, y and z, which may be identical or different, each represents 0 or 1,
- Y represents CO or CONH,
- Z represents a group Z₉ or Z'₉: 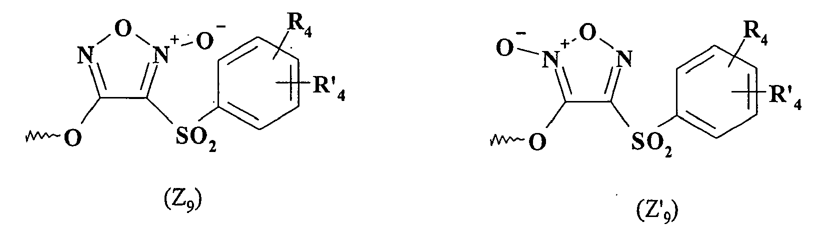 wherein R₄ and R'₄, which may be identical or different, each represents a hydrogen or halogen atom or a linear or branched C₁-C₆alkyl, trifluoromethyl or trifluoromethoxy group.

6. Process for the synthesis of the compounds of formula (I) according to claim 1, wherein compound A is reacted with compound B in an amount at least equal to 1 equivalent of compound A.

7. Pharmaceutical composition comprising as active ingredient a compound of formula (I) according to any one of claims 1 to 5 in combination with one or more pharmaceutically acceptable, inert, non-toxic excipients or carriers.

8. Pharmaceutical composition according to claim 7 for use in the treatment of cardiovascular pathologies.

9. Pharmaceutical composition according to claim 8 for use in the treatment of arterial hypertension and the vascular and renal sequelae thereof, systolic hypertension, peripheral vascular diseases, atherosclerosis, restenosis, cardiac insufficiency, thrombosis and any thromboembolic events, stable or unstable angina pectoris, cerebral vascular accidents, coronary accidents, myocardial infarction, vascular remodelling, diabetes and the vascular and renal sequelae thereof, complications associated with cardiovascular surgical operations, or endothelial dysfunction.

10. Pharmaceutical composition according to claim 7 for use in the treatment of pathologies involving inflammation and oxidative stress.

## Patentansprüche

1. Verbindungen der Formel (I):
A • B (I)
in der A Perindopril oder Perindoprilat bedeutet und
B eine Verbindung ist, die mindestens eine in ein Salz überführbare Säurefunktion aufweist, wiedergegeben durch die folgende Formel (III):
X-(Ak₁)ₓ(Y)_{y}-(Ak₂)_{z}-Z (III)
in der:
- X eine Gruppe CO₂H, SO₃H oder P(O)(OH)₂ bedeutet,
- Ak₁ und Ak₂, die identisch oder verschieden sind, jeweils eine geradkettige oder verzweigte, gesättigte oder ungesättigte C₁-C₈-Alkylengruppe bedeuten, in der ein oder mehrere der Kohlenstoffatome durch ein Sauerstoffatom, Schwefelatom, Stickstoffatom oder eine Gruppe SO₂ ersetzt sein können, wobei die Alkylengruppe gegebenenfalls durch eine oder mehrere Gruppen ausgewählt aus CO₂H, SO₃H, Hydroxy und Amino substituiert ist,
- x, y und z, die identisch oder verschieden sind, jeweils 0 oder 1 bedeuten,
- Y CO oder CONH bedeutet,
- Z eine NO-Donorgruppe bedeutet ausgewählt aus den folgenden Gruppen: 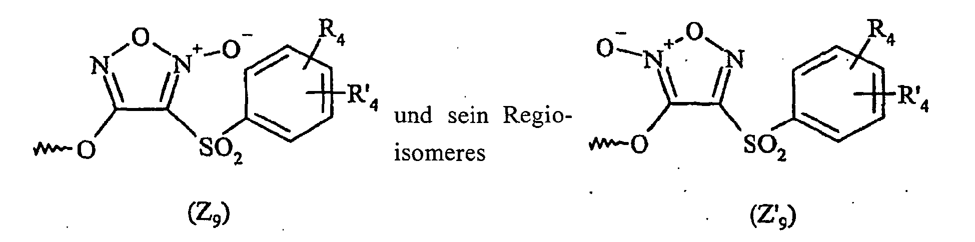 worin R₄ und R'₄, die identisch oder verschieden sind, jeweils ein Wasserstoffatom oder ein Halogenatom oder eine geradkettige oder verzweigte C₁-C₆-Alkylgruppe, Trifluormethylgruppe oder Trifluormethoxygruppe bedeuten,
wobei es sich versteht, dass die Bindung zwischen A und B eine ionische ist.

2. Verbindungen der Formel (I) nach Anspruch 1, worin die Verbindung der Formel (III) ausgewählt ist aus den Verbindungen der folgenden Formeln:
HO₂C-(Ak₁)ₓ-Z (IIIa)
HO₂C-CH=CH-(Ak₃)ₓ-Z (IIIb) 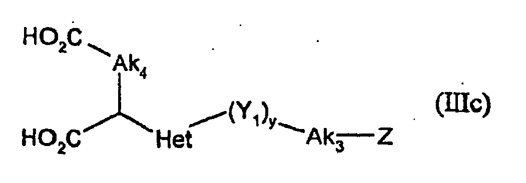 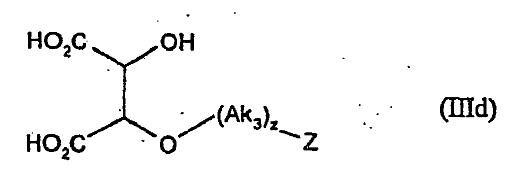 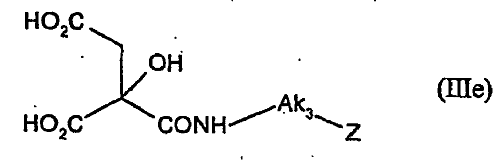 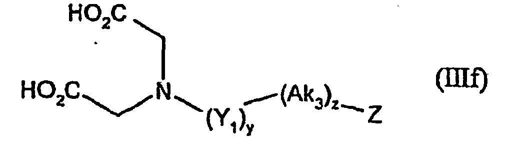 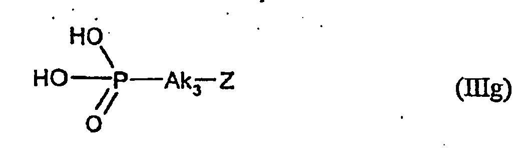 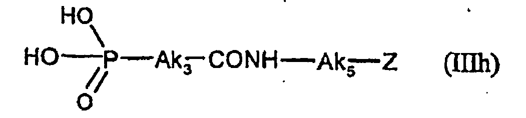
HO₃S-Ak₆-CONH-Ak₅-Z (IIIi) 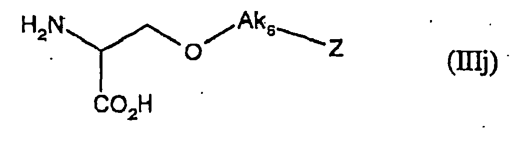 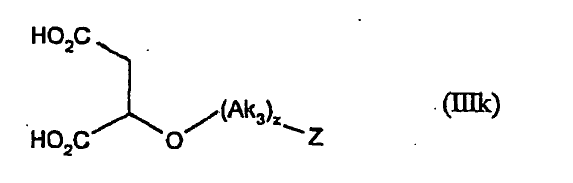 worin:
Ak₁, x, y, z und Z die in Anspruch 1 angegebenen Bedeutungen besitzen,
Ak₃ und Ak₅, die identisch oder verschieden sind, jeweils eine geradkettige oder verzweigte, gesättigte C₁-C₆-Alkylengruppe bedeuten, in der ein oder mehrere der Kohlenstoffatome durch ein Sauerstoffatom, Schwefelatom, Stickstoffatom oder eine Gruppe SO₂ ersetzt sein können,
Ak₄ eine geradkettige oder verzweigte, gesättigte C₁-C₃-Alkylengruppe bedeutet, in der eines der Kohlenstoffatome durch ein Sauerstoffatom, Schwefelatom, Stickstoffatom oder eine Gruppe SO₂ ersetzt sein kann,
Ak₆ eine geradkettige oder verzweigte, gesättigte C₂-C₅-Alkylengruppe bedeutet, in der eines der Kohlenstoffatome durch ein Sauerstoffatom, Schwefelatom, Stickstoffatom oder eine Gruppe SO₂ ersetzt sein kann,
Het O oder NH bedeutet und
Y₁ eine Carbonygruppe bedeutet.

3. Verbindungen der Formel (I) nach einem der Ansprüche 1 oder 2, worin B aus den folgenden Verbindungen ausgewählt ist:

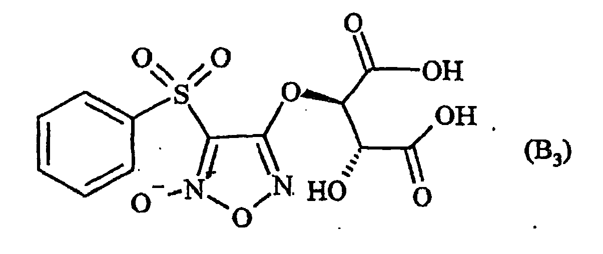

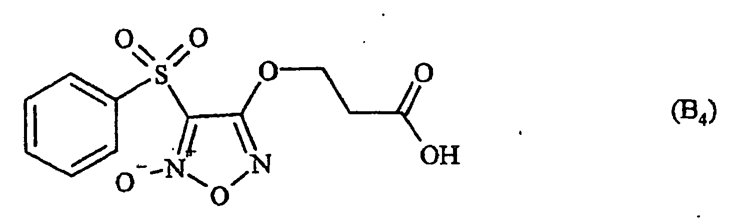

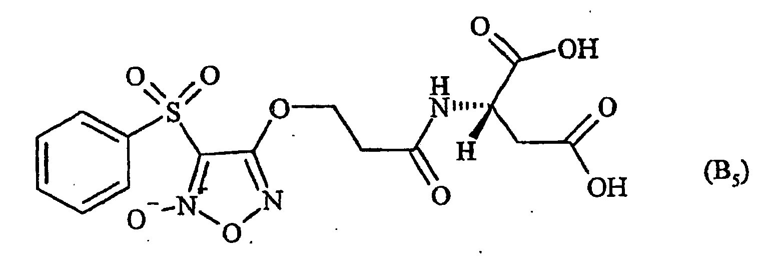

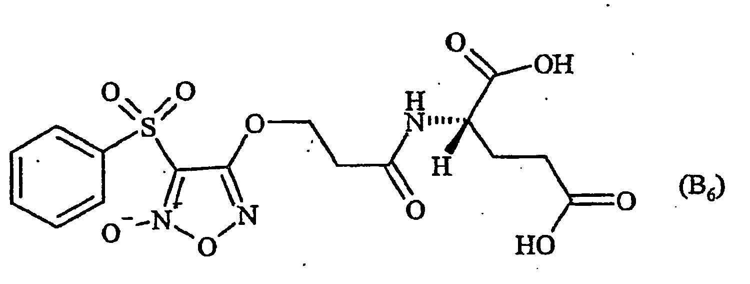

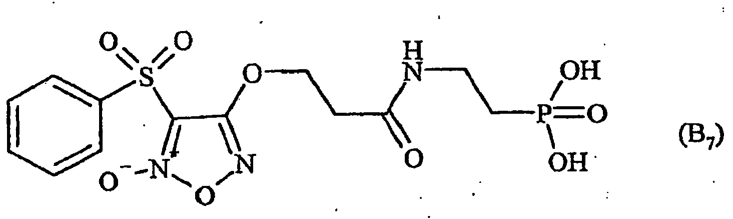

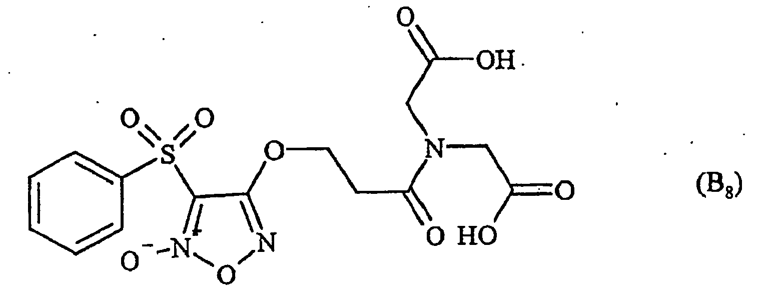

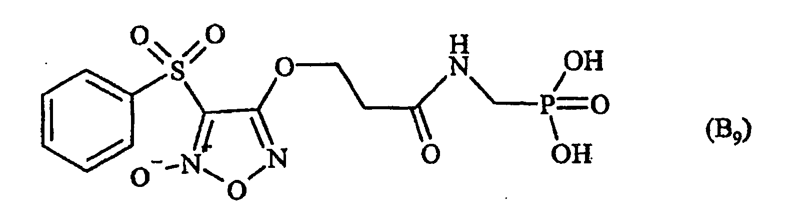

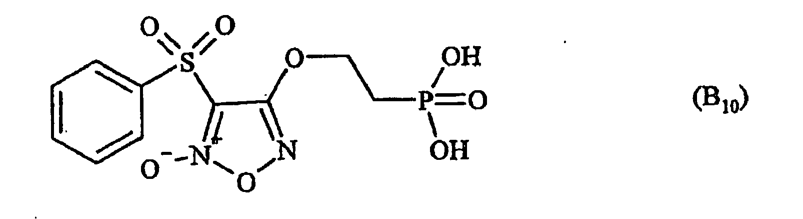

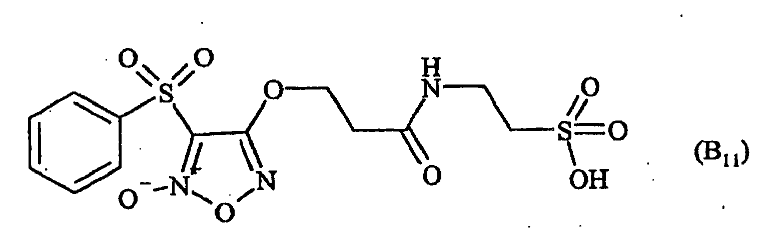

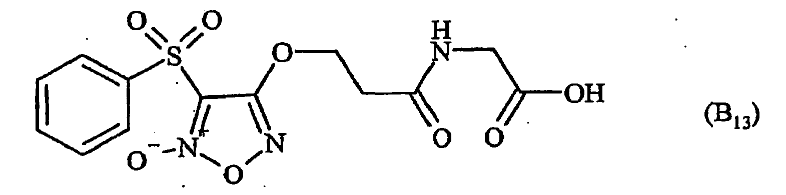

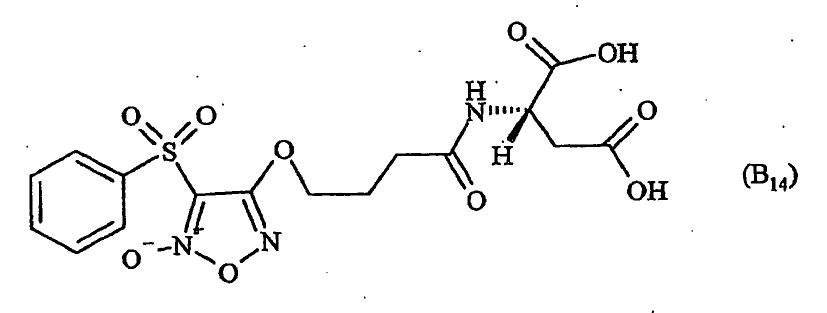

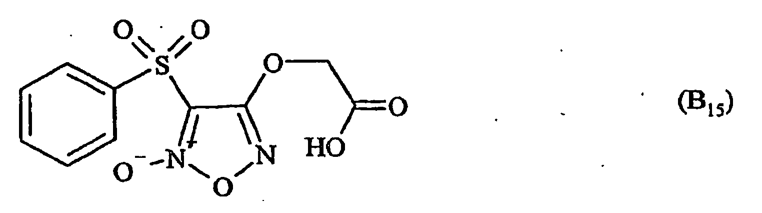

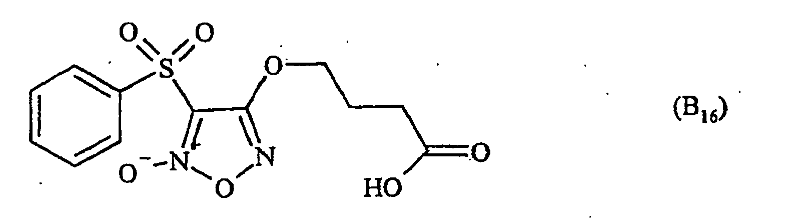

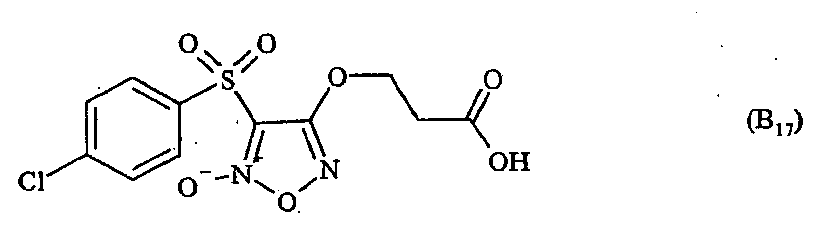

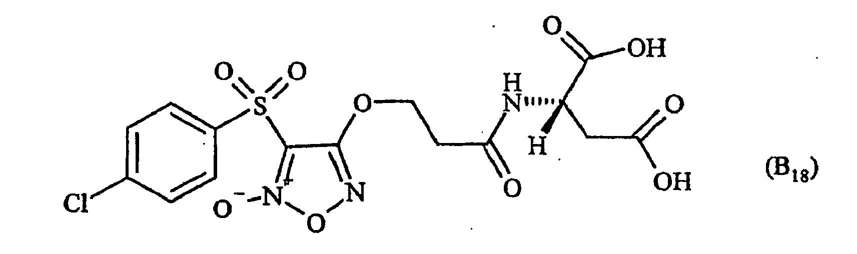

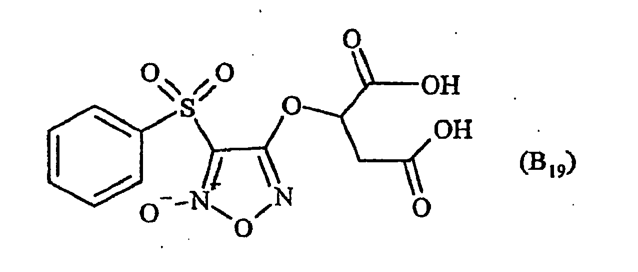

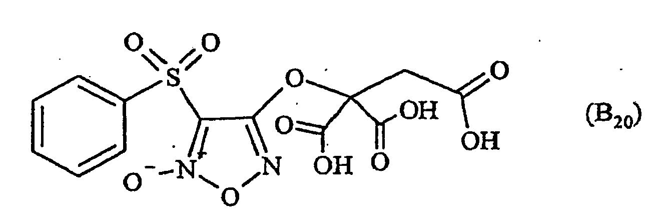

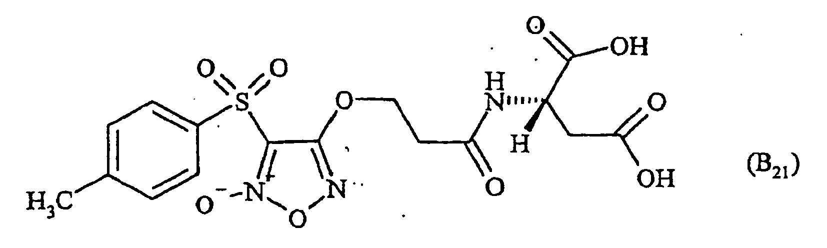

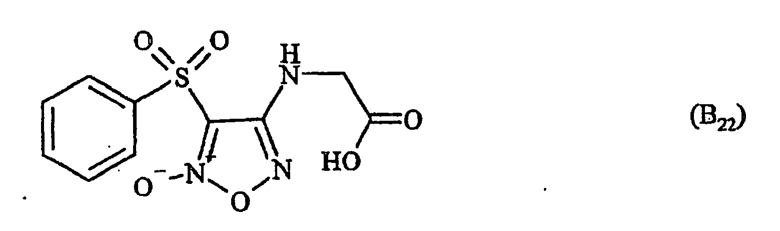

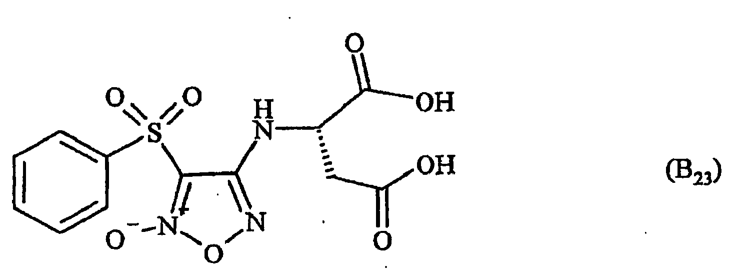

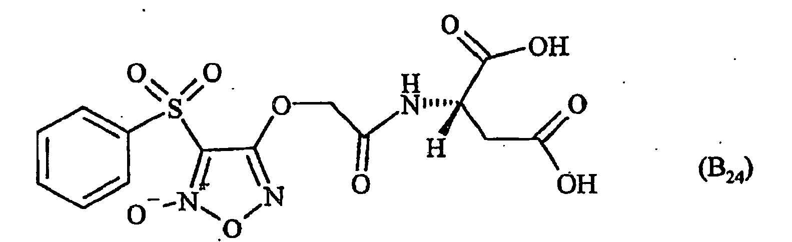

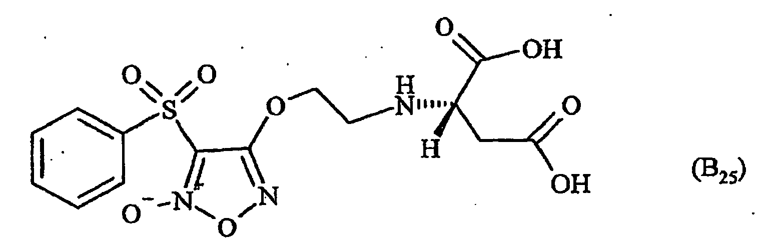

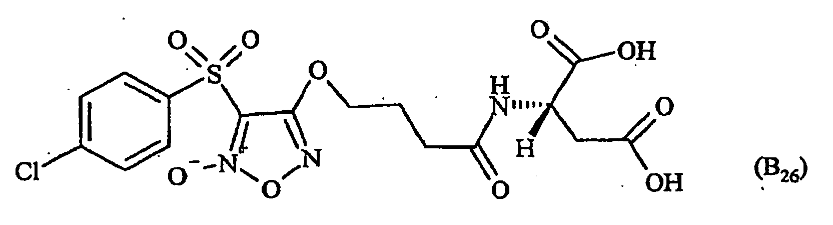

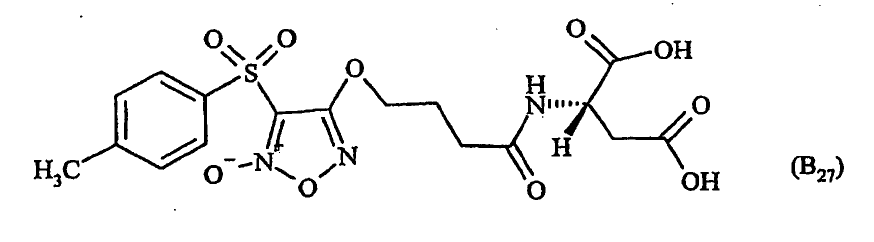

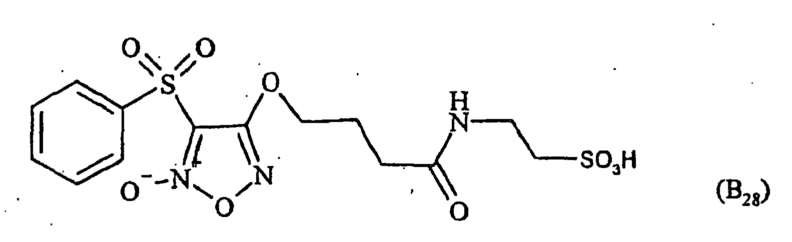

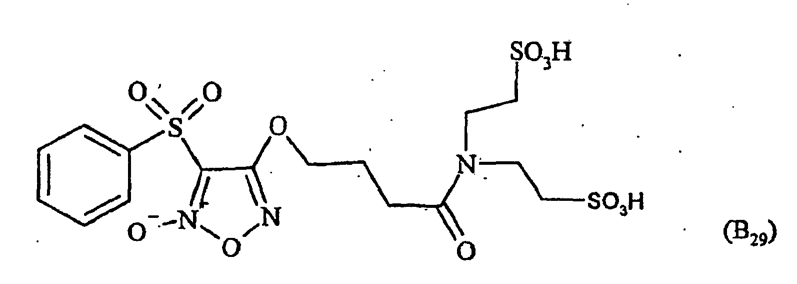

4. Verbindungen der Formel (I) nach Anspruch 1, ausgewählt aus den folgenden Verbindungen:
- (2*S*)-2-[(3-{[5-Oxido-4-(phenylsulfonyl)-1,2,5-oxadiazol-3-yl]-oxy}-propionyl)-amino]-bernsteinsäure - (2S,3aS,7aS)-1-[(2S)-2-{[(1S)-1-(Ethoxycarbonyl)-butyl]-amino}-propionyl]-octahydro-1H-indol-2-carbonsäure (1:1);
- (2*S*)-2-[(3-{[5-Oxido-4-(phenylsulfonyl)-1,2,5-oxadiazol-3-yl]-oxy}-propionyl)-amino]-pentandisäure - (2*S*,3a*S*,7a*S*)-1-[(2*S*)-2-{[(1*S*)-1-(Ethoxycarbonyl)-butyl]-amino}-propionyl]-octahydro-1*H-*indol-2-carbonsäure (1:1);
- 2-[(3-{[5-Oxido-4-(phenylsulfonyl)-1,2,5-oxadiazol-3-yl]-oxy}-propionyl)-amino]-ethansulfonsäure - (2*S*,3a*S*,7a*S*)-1-[(2*S*)-2-{[(1*S*)-1-(Ethoxycarbonyl)-butyl]-amino}-propionyl]-octahydro-1*H-*indol-2-carbonsäure (1:1);
- (2*S*,3a*S*,7a*S*)-1-[(2*S*)-2-{[(1*S*)-1-(Ethoxycarbonyl)-butyl]-amino}-propionyl]-octa-hydro-1*H-*indol-2-carbonsäure - {[(3-{[5-Oxido-4-(phenylsulfonyl)-1,2,5-oxadiazol-3-yl]-oxy}-propionyl)-amino]-methyl}-phosphonsäure (1:1);
- (2*S*,3a*S*,7a*S*)-1-[(2*S*)-2-{[(1*S*)-1-(Ethoxycarbonyl)-butyl]-amino}-propionyl]-octa-hydro-1*H-*indol-2-carbonsäure - 2,2'-[(3-{[5-Oxido-4-(phenylsulfonyl)-1,2,5-oxadiazol-3-yl]-oxy}-propionyl)-imino]-diessigsäure (1:1);
- (2*S*,3a*S*,7a*S*)-1-[(2*S*)-2-{[(1*S*)-1-(Ethoxycarbonyl)-butyl]-amino}-propionyl]-octahydro-1*H-*indol-2-carbonsäure- {2-[(3-{[5-Oxido-4-(phenylsulfonyl)-1,2,5-oxadiazol-3-yl]-oxy}-propionyl)-amino]-ethyl}-phosphonsäure (1:1);
- (2*S*,3a*S*,7a*S*)-1-[(2*S*)-2-{[(1*S*)-1-(Ethoxycarbonyl)-butyl]-amino}-propionyl]-octahydro-1*H-*indol-2-carbonsäure - (2-{[5-Oxido-4-(phenylsulfonyl)-1,2,5-oxadiazol-3-yl]-oxy}-ethyl}-phosphonsäure (1:1);
- (2S)-2-[(4-{[5-Oxido-4-(phenylsulfonyl)-1,2,5-oxadiazol-3-yl]-oxy}-butanoyl)-amino]-bernsteinsäure - (2S,3aS,7aS)-1-[(2S)-2-{[(1S)-1-(Ethoxycarbonyl)-butyl]-amino}-propionyl]-octahydro-1H-indol-2-carbonsäure (1:1);
- (2S)-2-{[3-({4-[(4-Chlorphenyl)-sulfonyl]-5-oxido-1,2,5-oxadiazol-3-yl}-oxy)-propionyl]-amino}-bernsteinsäure - (2S,3aS,7aS)-1-[(2S)-2-{[(1S)-1-(Ethoxycarbonyl)-butyl]-amino}-propionyl]-octahydro-1H-indol-2-carbonsäure (1:1);
- (2S)-2-{[3-({4-[(4-Methylphenyl)-sulfonyl]-5-oxido-1,2,5-oxadiazol-3-yl}-oxy)-propionyl]-amino}-bernsteinsäure - (2S,3aS,7aS)-1-[(2S)-2-{[(1S)-1-(Ethoxycarbonyl)-butyl]-amino}-propionyl]-octahydro-1*H-*indol-2-carbonsäure (1:1);
- (2S)-2-{[3-({4-[(4-Methylphenyl)-sulfonyl]-5-oxido-1,2,5-oxadiazol-3-yl}-oxy)-butanoyl]-amino}-bernsteinsäure - (2S,3aS,7aS)-1-[(2S)-2-{[(1S)-1-(Ethoxycarbonyl)-butyl]-amino}-propionyl]-octahydro-1*H-*indol-2-carbonsäure (1:1).

5. Verbindungen der Formel (III):
X-(Ak₁)ₓ-(Y)_{y}-(Ak₂)_{z}-Z (III)
in der:
- X eine Gruppe CO₂H, SO₃H oder P(O)(OH)₂ bedeutet,
- Ak₁ und Ak₂, die identisch oder verschieden sind, jeweils eine geradkettige oder verzweigte, gesättigte oder ungesättigte C₁-C₈-Alkylengruppe bedeuten, in der eines oder mehrere der Kohlenstoffatome durch ein Sauerstoffatom, Schwefelatom, Stickstoffatom oder eine Gruppe SO₂ ersetzt sein können, wobei die Alkylengruppe gegebenenfalls durch eine oder mehrere Gruppen ausgewählt aus Carboxy, SO₃H, Hydroxy und Amino substituiert ist,
- x, y und z, die identisch oder verschieden sind, jeweils 0 oder 1 bedeuten,
- Y CO oder CONH bedeutet,
- Z eine Gruppe Z₉ oder Z'₉ bedeutet: 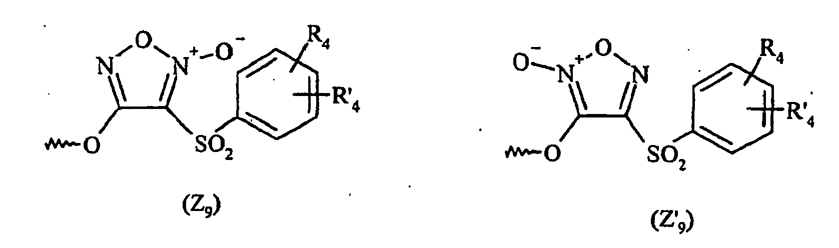 worin R₄ und R'₄, die identisch oder verschieden sind, jeweils ein Wasserstoffatom oder Halogenatom oder eine geradkettige oder verzweigte C₁-C₆-Alkylgruppe, Trifluormethylgruppe oder Trifluormethoxygruppe bedeuten.

6. Verfahren zur Synthese der Verbindungen der Formel (I) nach Anspruch 1, worin die Verbindung A mit der Verbindung B in einer Menge, die mindestens einem Äquivalent der Verbindung A entspricht, umgesetzt wird.

7. Pharmazeutische Zusammensetzung enthaltend als Wirkstoff eine Verbindung der Formel (I) nach einem der Ansprüche 1 bis 5 in Kombination mit einem oder mehreren inerten, nichttoxischen, pharmazeutisch annehmbaren Hilfsstoffen oder Trägermaterialien.

8. Pharmazeutische Zusammensetzung nach Anspruch 7, nützlich zur Behandlung von kardiovaskulären Erkrankungen.

9. Pharmazeutische Zusammensetzung nach Anspruch 8, nützlich zur Behandlung der arteriellen Hypertension und seinen Folgen für die Gefäße und die Nieren, der systolischen Hypertension, peripheren Gefäßerkrankungen, Atherosklerose, Restenose, Herzinsuffizienz, Thrombose und sämtlichen thromboembolischen Ereignissen, stabiler oder instabiler Angina pectoris, Gehirngefäßvorfällen (Schlaganfällen), Koronaranfällen, Myokardinfarkt, Gefäßveränderungen, Diabetes und seinen Konsequenzen für die Gefäße und die Nieren, Komplikationen, die mit kardiovaskulären chirurgischen Eingriffen verknüpft sind, oder der endothelialen Dysfunktion.

10. Pharmazeutische Zusammensetzung nach Anspruch 7, nützlich zur Behandlung von Erkrankungen, die Entzündungen und oxidativen Stress umfassen.
